(19) Europäisches Patentamt
European Patent Office
Office européen des brevets

(11) **EP 4 697 337 A1**

(12) **EUROPÄISCHE PATENTANMELDUNG**

(43) Veröffentlichungstag:
**18.02.2026  Patentblatt 2026/08**

(21) Anmeldenummer: **25186613.3**

(22) Anmeldetag: **01.07.2025**

(51) Internationale Patentklassifikation (IPC):
*G16C 60/00* (2019.01)    *G16C 20/30* (2019.01)
*G16C 20/70* (2019.01)

(52) Gemeinsame Patentklassifikation (CPC):
**G16C 60/00;** G06N 3/00; G16C 20/30; G16C 20/70

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**
Benannte Erstreckungsstaaten:
**BA**
Benannte Validierungsstaaten:
**GE KH LA MA MD TN**

(30) Priorität: **14.08.2024  DE 102024123245**

(71) Anmelder: **Gebrüder Dorfner GmbH & Co. KG**
**92242 Hirschau (DE)**

(72) Erfinder:
• **Mondan, Mirko**
**92224 Amberg (DE)**
• **van Herzele, Peter**
**92637 Weiden in der Oberpfalz (DE)**
• **Grünwald, Florian**
**92253 Schnaittenbach (DE)**
• **Drum, Oliver**
**92253 Schnaittenbach (DE)**

(74) Vertreter: **Hannke, Christian**
**Hannke Bittner & Partner**
**Patent- und Rechtsanwälte mbB**
**Prüfeninger Straße 1**
**93049 Regensburg (DE)**

(54) **VERFAHREN UND EIGENSCHAFTS-ERMITTLUNGSVORRICHTUNG ZUR ERMITTLUNG VON BESCHICHTUNGSZUSAMMENSETZUNGSEIGENSCHAFTEN**

(57)    Die vorliegende Erfindung betrifft ein Verfahren zur, insbesondere prädiktiven, Ermittlung wenigstens einer physiko-chemischen Beschichtungszusammensetzungseigenschaft $(Y_Z)$ einer ersten Beschichtungszusammensetzung $(X_Z)$, welches eine erste Beschichtungszusammensetzungskomponente $(A_Z)$ und wenigstens eine weitere, von der ersten Beschichtungszusammensetzungskomponente $(A_Z)$ verschiedene Beschichtungszusammensetzungskomponente $(B_Z)$ umfasst, mit den Schritten:
- Erfassung eines ersten Analysedatensatzes $(X_2)$ zu einer zweiten Beschichtungszusammensetzung,
- Erfassung mindestens eines weiteren Analysedatensatzes $(X_3, ... X_n)$, zu mindestens einer dritten Beschichtungszusammensetzung, und
- Computer-implementierte Ermittlung von wenigstens einer für die Beschichtungszusammensetzungseigenschaft $(Y_Z, Z_Z,...)$ der ersten Beschichtungszusammensetzung $(X_Z)$ charakteristischen physiko-chemischen Eigenschaftsgröße auf Grundlage des Analysedatensatzes $(X_2)$ zu der zweiten Beschichtungszusammensetzung und des mindestens einen weiteren Analysedatensatzes $(X_3, ... X_n)$ zu der mindestens einen dritten Beschichtungszusammensetzung.
    Außerdem betrifft der Erfindung ein Computer-implementiertes Verfahren zur Erzeugung eines trainierten Beschichtungszusammensetzungseigenschafts-Ermittlungsmodells maschinellen Lernens (6) zur Ermittlung wenigstens einer physiko-chemischen Beschichtungszusammensetzungseigenschaft $(Y_Z, Z_Z,...)$ einer Beschichtungszusammensetzung auf Basis einer Verwendung von Analysedatensätzen $(X_2 - X_n)$, sowie eine Beschichtungszusammensetzungseigenschafts-Ermittlungsvorrichtung.

Fig. 1

**Beschreibung**

Die vorliegende Erfindung betrifft ein Verfahren zur Ermittlung von Beschichtungszusammensetzungseigenschaften. Weiterhin betrifft die Erfindung eine Eigenschafts-Ermittlungsvorrichtung zur, insbesondere prädiktiven, Ermittlung von Beschichtungszusammensetzungseigenschaften. Weiterhin bezieht sich die Erfindung auf ein Verfahren zur Erzeugung eines (trainierten) Beschichtungszusammensetzungseigenschafts-Ermittlungsmodells maschinellen Lernens zur Ermittlung wenigstens einer Beschichtungszusammensetzungseigenschaft einer Beschichtungszusammensetzung und eine Verwendung des Modells zur Bestimmung bzw. zur Ermittlung wenigstens einer Beschichtungszusammensetzungseigenschaft.

Aus dem Stand der Technik sind eine Vielzahl verschiedener Beschichtungen und Beschichtungszusammensetzungen bekannt, welche für die jeweiligen Aufgaben und Anwendungsszenarien angepasst sind.

So sind beispielsweise Anstrichstoffe bekannt. Dabei handelt es sich üblicherweise um flüssige bis pastenförmige und seltener pulverförmige Stoffe oder Gemische, die auf Oberflächen aufgetragen einen physikalisch trocknenden oder chemisch härtenden Anstrich ergeben. Nach DIN 55945 ist ein Anstrichstoff definiert als ein flüssig bis pastenförmiger Beschichtungsstoff, der vorwiegend durch Streichen oder Rollen aufgetragen wird.

Wesentliche Bestandteile eines Anstrichstoffs sind oftmals ein Bindemittel, ein Farbmittel (Pigment oder löslicher Farbstoff), ein oder mehrere Füllstoffe, ein Lösungsmittel, sowie ein eventueller Zusatzstoff wie Verdickungsmittel, Dispergiermittel, Entschäumer und Konservierungsmittel. Als Anstrichfarben oder Malfarben werden Anstrichmittel verstanden, die Pigmente enthalten.

Anstrichstoffe werden oftmals nach ihren filmbildenden Bindemitteln klassifiziert. Diese wiederum lassen sich vorrangig in organische und anorganische Bindemittel unterteilen und ergeben die Produktklassen Lack, Lasur und Ölfarben; Dispersionsfarben; Kalkfarben; Silikatfarben; und Flüssig-Putze.

Einen hohen Marktanteil der Anstrichstoffe machen Anstriche mit Dispersionsfarbe aus. Dispersionsfarben sind üblicherweise zäh- bis dünnflüssige Anstrichstoffe. Sie bestehen aus einer Dispersion (meistens einer Suspension) aus Lösemittel, Binde- und Lösungsmitteln, Pigmenten sowie Füllstoffe und Hilfsmittel oder Additive. Auch wenn es sich in diesem allgemeinen Sinn bei der Mehrzahl der flüssigen Anstriche (Lacke, Farben) um Dispersionen handelt, wird der Begriff "Dispersionsfarbe" im engeren und umgangssprachlichen Sinn meist als eine handelsübliche Wandfarbe auf Wasserbasis verstanden. Hochwertigere Farben werden oft als Acryl- oder Kunstharzdispersionfarben bezeichnet.

Daneben gibt es auf dem Markt noch ähnlich aufgebaute wasserhaltige Wandfarben, die statt synthetischen und mineralölhaltigen Zutaten vor allem Pflanzenöle verwenden und als Naturdispersionsfarben bezeichnet werden.

Kunstharzdispersionsanstriche (auch Kunststoffdispersionsanstriche, -farben, oder -dispersionen) sind Wandanstriche, die in der Regel aus einer Dispersion von Kunstharz und Wasser bestehen. Für den Innenbereich sind sie Qualitätsmerkmale in der EN 13300 genormt, solche für den Außenbereich in der EN 1062. Hauptbestandteile sind typischerweise Wasser als Lösungsmittel, aus fossilen Monomeren und/oder biobasierten Monomeren gewonnene Kunstharze (meist Acrylharze) oder ähnliche Kunststoffe (beispielsweise Polyvinylacetat) als Bindemittel, und als farbgebende Farbstoffe oder Pigmente. Kunstharzdispersionswandfarben mit besonders hoher Wasserfestigkeit, für den Außen- und Fassadenbereich sowie für Feuchtbereiche im Hausinneren enthalten einen erhöhten Anteil an Kunstharz. Kunstharzdispersionsfarben mit dekorativen Zusätzen wie Glitter werden ebenfalls als Latexfarben bezeichnet.

Neben flüssiger Kunstharzdispersionsfarbe gibt es thixotrope (kompakte) Anstriche. Durch ihre Thixotropie sollen sie beim Streichen weniger klecksen und spritzen. Derartige, als Kompaktfarben bezeichneten Anstrichstoffe haben an Marktbedeutung verloren, da die meisten Dispersionsfarben mittlerweile tropfgehemmt (leicht geleeartig) eingestellt sind und sich im Gegensatz zu Kompaktfarben wesentlich leichter applizieren lassen.

Für Farben und Anstriche existieren eine Reihe von Normen und Prüfkriterien, mit denen versucht wird, den Anwendern Hinweise dafür zu geben, welche Farbe für welche Anwendung geeignet ist. So wird beispielsweise durch die Norm EN 13300 eine qualitative Einteilung von Wand- und Deckenfarben nach vorgesehener Anwendung und Bindemitteltyp vorgenommen. Dabei werden unter anderem folgende Kriterien zur Unterscheidung genutzt:

- Kontrastverhältnis (Deckvermögen),
- Glanz / Reflexionswert,
- Maximale Korngröße, und
- Nassabriebbeständigkeit

Die auf dem Markt erhältlichen Farbzusammensetzungen sind dementsprechend vielfältig. Die erhältlichen Farbzusammensetzungen sind in Abhängigkeit des jeweiligen Anwendungsfalls auszuwählen. Für die Hersteller von Farbzusammensetzungen besteht die Notwendigkeit, für einen vorgegebenen Anwendungsfall eine möglichst geeignete Farbzusammensetzung bereitstellen zu können. Dies wurde bisher dadurch gewährleistet, dass Farbzusammensetzung bereitgestellt wurden, die für möglichst viele verschiedene Anwendungsfälle einsetzbar sind.

Dies hat jedoch den Nachteil, dass diese Farbzusammensetzungen - um auch für andere Anwendungen geeignet sein zu können - meist nicht optimal für eine einzelne Anwendung geeignet sind. Außerdem ist der Einsatz von meist kostenintensiven Rohstoffen erhöht, da derartige Farbzusammensetzungen auch Inhaltsstoffe enthalten oder in erhöhter Menge enthalten, die für die vorgesehene Anwendung nicht oder zumindest nicht in dieser Menge notwendig wären.

Zur Entwicklung neuer Farbzusammensetzungen wurden bislang eine Vielzahl von Farbzusammensetzungen im Labormaßstab hergestellt und deren Eigenschaften in teilweise aufwändigen und langwierigen Laboruntersuchungen gemessen.

Außerdem wurden in der Vergangenheit Untersuchungen durchgeführt, die die Auswirkungen einzelner Inhaltsstoffe auf verschiedene Parameter der Farbzusammensetzung ermitteln. Die Ermittlung des Effekts einzelner Bestandteile auf die Eigenschaften der gesamten Farbzusammensetzung ist jedoch zeit- und kostenintensiv. Außerdem sind derartige Untersuchungen nicht sehr zuverlässig, da bei diesen Testreihen Synergieeffekte zwischen verschiedenen Komponenten einer Farbzusammensetzung eventuell nicht korrekt abgebildet werden. Sollten auch derartige Effekte berücksichtigt werden, wäre eine noch mehrfach größere Anzahl von Laboruntersuchungen notwendig, die das Kosten/Nutzen-Verhältnis in den meisten Fällen sprengen würde. Zudem würde eine solche Untersuchung in den meisten Fällen zu einer enormen Datenmenge führen, deren Auswertung ebenfalls einen enormen Zeitaufwand hat.

Bisher wurde daher versucht, einige wenige Bestandteile von Farbzusammensetzungen zu identifizieren und deren gegenseitige positive oder negative Beeinflussung innerhalb einer Farbzusammensetzung zu ermitteln. Diese Untersuchungen liefern jedoch kein zufriedenstellendes Ergebnis, da einerseits eine Beschränkung auf eine geringe Anzahl von Komponenten notwendig ist und andererseits dennoch eine große Datenmenge erzeugt wird und ausgewertet werden muss.

Der vorliegenden Erfindung liegt daher die Aufgabe zugrunde, die aus dem Stand der Technik bekannten Nachteile zu überwinden und ein Verfahren sowie eine Eigenschaftsermittlungsvorrichtung zur Ermittlung von Beschichtungszusammensetzungseigenschaften bereitzustellen. Diese sollte möglichst eine verlässliche und gleichwohl schnelle und möglichst wenig rechenintensive Möglichkeit der Vorhersage von Beschichtungszusammensetzungseigenschaften bieten.

Diese Aufgabe wird erfindungsgemäß durch die Gegenstände der unabhängigen Ansprüche gelöst. Vorteilhafte Ausführungsformen und Weiterbildungen der Erfindung sind Gegenstand der abhängigen Ansprüche.

Bei einem erfindungsgemäßen Verfahren zur, insbesondere prädiktiven, Ermittlung wenigstens einer physiko-chemischen Beschichtungszusammensetzungseigenschaft einer ersten Beschichtungszusammensetzung, umfasst diese zumindest eine erste Beschichtungszusammensetzungskomponente und wenigstens eine weitere, von der ersten Beschichtungszusammensetzungskomponente (A1) verschiedene Beschichtungszusammensetzungskomponente (B2).

Bevorzugt umfasst die ersten Beschichtungszusammensetzung eine Vielzahl von Beschichtungszusammensetzungskomponenten, wobei beispielsweise mehr als zwei, bevorzugt mehr als drei, bevorzugt mehr als fünf, bevorzugt mehr als acht, bevorzugt mehr als zehn und besonders bevorzugt mehr als 15 Beschichtungszusammensetzungskomponenten in der ersten Beschichtungszusammensetzung enthalten sein können bzw. zu der ersten Beschichtungszusammensetzung umgesetzt sein können. Unter den Beschichtungszusammensetzungskomponenten sind insbesondere Substanzen oder Abmischungen zu verstehen, die aktiv zusammengeführt werden (können), um die Beschichtungszusammensetzung zu erhalten. Eventuell in einer oder mehreren der Beschichtungszusammensetzungskomponenten enthaltenen Verunreinigungen bleiben bei dieser Betrachtung unberücksichtigt. Werden auch eventuell enthaltene Verunreinigungen als Beschichtungszusammensetzungskomponenten betrachtet kann deren Anzahl in der Beschichtungszusammensetzung auch deutlich über 50 oder sogar > 100 liegen, da oftmals die Beschichtungszusammensetzungskomponenten lediglich in technischer Reinheit und/oder beispielsweise einer Reinheit ≥ 99 Gewichtsprozent eingesetzt werden.

Bei der ersten Beschichtungszusammensetzung handelt es sich vorzugsweise um eine Beschichtungszusammensetzung, die ausgewählt ist aus einer Gruppe, die einen Anstrichstoff, Lack, Lasur, Ölfarbe, Dispersionsfarbe, Kalkfarben, Silikatfarbe, Flüssig-Putz, Acrylharzdispersionfarbe, Kunstharzdispersionfarbe, Wandfarbe und thixotropen Anstrich umfasst.

In dem erfindungsgemäßen Verfahren wird ein erster Analysedatensatzes zu einer zweiten Beschichtungszusammensetzung erfasst. Der erste Analysedatensatz umfasst wenigstens eine Analysegröße und eine Anteilsgröße einer ersten Beschichtungszusammensetzungskomponente. Bevorzugt umfasst der erste Analysedatensatz aber eine Vielzahl von Anteilsgrößen von weiteren Beschichtungszusammensetzungskomponenten.

Außerdem ist bei dem erfindungsgemäßen Verfahren vorgesehen, dass mindestens ein weiterer Analysedatensatzes zu mindestens einer dritten Beschichtungszusammensetzung erfasst wird. Der zweite Analysedatensatz umfasst wenigstens eine Analysegröße und eine Anteilsgröße einer ersten Beschichtungszusammensetzungskomponente. Bevorzugt umfasst der zweite Analysedatensatz jedoch eine Vielzahl von Anteilsgrößen von weiteren Beschichtungszusammensetzungskomponenten.

Die Analysegröße ist dabei jeweils charakteristisch für wenigstens eine Beschichtungszusammensetzungseigenschaft der jeweiligen Beschichtungszusammensetzung. Beispielsweise könnte es sich bei der Beschichtungszusammensetzungseigenschaft um eine Nassabriebbeständigkeit, ein Deckvermögen , einen Farbwert, eine Viskosität, einen Glanz oder sonstige Eigenschaft der Beschichtungszusammensetzung handeln.

Die Anteilsgröße ist bei dem erfindungsgemäßen Verfahren jeweils charakteristisch für einen Mengenanteil der in der jeweiligen Beschichtungszusammensetzung befindlichen ersten und/oder weiteren Beschichtungszusammensetzungskomponente.

Unter einer "Erfassung eines Analysedatensatzes" wird insbesondere ein Empfangen des über eine Kommunikationseinrichtung übermittelten Analysedatensatzes und/oder ein Abrufen des Analysedatensatzes von einer, insbesondere nichtflüchtigen, Speichereinrichtung (etwa einer externen Speichereinrichtung wie beispielsweise einem externen (Backend-)Server) durch eine, insbesondere Prozessor-basierte und bevorzugt nachfolgend näher beschriebene, Beschichtungszusammensetzungseigenschafts-Ermittlungsvorrichtung verstanden, so dass (durch das Erfassen) bevorzugt ein Analysedatensatz (oder mehrere Analysedatensätze) zur Datenverarbeitung des Analysedatensatzes bereitgestellt wird.

Daneben kann gemäß einer weiteren Ausführungsform unter "Erfassung eines Analysedatensatzes" zusätzlich oder alternativ auch ein Ermitteln und/oder Erzeugen des Analysedatensatzes zu verstehen sein. Dies könnte durch rechnerische Ermittlung, etwa durch Interpolation und/oder Extrapolation vorgegebener Datenwerte (etwa wenigstens einer oder mehrerer ähnlicher Beschichtungszusammensetzungen) und/oder durch experimentelles Erzeugen bzw. experimentelles Bestimmen und/oder experimentelles Messen (von Werten) des Analysedatensatzes erfolgen.

Wie oben dargelegt, enthält ein Analysedatensatz Daten zu Analysegröße/n, Anteilsgröße/n und Beschichtungszusammensetzungskomponente/n. Ein im Analysedatensatz enthaltenes Datum steht dabei vorzugsweise eindeutig, insbesondere eineindeutig für eine Analysegröße, eine Anteilsgröße oder eine Beschichtungszusammensetzungskomponente. "Datum" soll im Rahmen dieser Beschreibung als Singular von "Daten", also als ein einzelner Eintrag in einem Datensatz und/oder ein einzelner Datenpunkt in einer Gruppe von Daten verstanden werden. Es kann sich bei einem solchen Datum um eine Zahl, eine Funktion, einen Buchstaben eine Buchstabenfolge oder Kombinationen davon handeln.

Im Folgenden wird nicht zwingend zwischen einem Datum, das eine Analysegröße, eine Anteilsgröße oder eine Beschichtungszusammensetzungskomponente in einem Analysedatensatz charakterisiert und dem tatsächlichen Wert der Analysegröße oder Anteilsgröße, beziehungsweise der tatsächlich in einer Beschichtungszusammensetzung enthaltenen Beschichtungszusammensetzungskomponente unterschieden. Sofern der Unterschied nicht explizit beschrieben ist, soll im Rahmen dieser Beschreibung der Erfindung eine Offenbarung zu einem Datum eines Analysedatensatz, das eine Analysegröße, eine Anteilsgröße oder eine Beschichtungszusammensetzungskomponente in einem Analysedatensatz charakterisiert auch als Offenbarung für den tatsächlichen Wert der Analysegröße oder Anteilsgröße, beziehungsweise der tatsächlich in einer Beschichtungszusammensetzung enthaltenen Beschichtungszusammensetzungskomponente gelten. Analog soll auch eine Beschreibung für einen tatsächlichen Wert der Analysegröße oder Anteilsgröße, beziehungsweise der tatsächlich in einer Beschichtungszusammensetzung enthaltenen Beschichtungszusammensetzungskomponente als Offenbarung für ein Datum in einem Analysedatensatz gelten, welches diese Analysegröße, Anteilsgröße oder Beschichtungszusammensetzungskomponente charakterisiert.

Analog soll im Rahmen dieser Beschreibung der Erfindung auch jede Beschreibung, die für einen Analysedatensatz gemacht ist als für die Beschichtungszusammensetzung als offenbart gelten, die durch diesen Analysedatensatz charakterisiert ist, sofern dies nicht explizit ausgeschlossen ist. Umgekehrt soll auch jedoch Offenbarung, die für eine Beschichtungszusammensetzung gegeben ist, als eine Offenbarung für einen Analysedatensatz gelten, der diese Beschichtungszusammensetzung charakterisiert.

Ebenso soll im Rahmen dieser Beschreibung der Erfindung auch jede Beschreibung, die für eine Beschichtungszusammensetzungseigenschaft beschrieben ist, auch als für eine Beschichtungszusammensetzungseigenschaftsgröße offenbart gelten und umgekehrt, da vorzugsweise eine in einem Datensatz enthaltene Beschichtungszusammensetzungseigenschaftsgröße eineindeutig, bevorzugt eineindeutig eine Beschichtungszusammensetzungseigenschaft charakterisiert.

Die in dem Analysedatensatz oder den Analysedatensätzen enthaltenen Daten zu Analysegröße/n, Anteilsgröße/n und Beschichtungszusammensetzungskomponente/n können (jeweils, bevorzugt unabhängig voneinander) rechnerisch ermittelt sein, oder durch eine (physiko-chemische) Analyse ermittelt sein. Vorzugsweise ist jedoch zumindest ein überwiegender Teil, vorzugsweise $\geq 75\,\%$, bevorzugt $\geq 90\,\%$, weiter bevorzugt $\geq 95\,\%$, meist bevorzugt $\geq 99\,\%$ der in dem Analysedatensatz oder den Analysedatensätzen enthaltenen Daten durch eine (physiko-chemische) Analyse ermittelt. Ein hoher Anteil an durch eine (physiko-chemische) Analyse ermittelten Daten in dem Analysedatensatz oder den Analysedatensätzen hat den Vorteil, dass diese üblicherweise weniger fehlerbehaftet sind als rechnerisch ermittelte Daten, da bei rechnerisch ermittelten Daten mögliche Fehler in den Daten, die die Berechnungsgrundlage bilden, mitgeschleift oder sogar vergrößert werden können.

Ein Analysedatensatz umfasst vorzugsweise jeweils Paare von Daten (Im Folgenden auch als Deskriptoren bezeichnet), die jeweils charakteristisch sind für eine Beschichtungszusammensetzungskomponente und deren Anteilsgröße in der durch diesen Datensatz charakterisierten Beschichtungszusammensetzung. Außerdem umfasst ein Analysedatensatz vorzugsweise mindestens ein Datum, das für eine Analysegröße charakteristisch ist. Bevorzugt ist jedoch, dass ein Analysedatensatz eine Vielzahl von für Analysegrößen charakteristische Daten enthält, wobei jedes Datum vorzugsweise für (bevorzugt genau) eine Analysegröße charakteristisch ist.

Insbesondere ist bevorzugt, dass ein Analysedatensatz (bevorzugt jeder oder zumindest nahezu jeder Analysedatensatz) mehrere der oben genannten Paare von Daten umfasst. Es hat sich gezeigt, dass eine Beschichtungszusammensetzung ausreichend genau charakterisiert sein kann, sofern mindestens zwei derartige Paare definiert sind. Eine so geringe Anzahl von Daten ist insbesondere dann ausreichend, wenn eine der durch ein solches Datum charakterisierten Beschichtungszusammensetzungskomponenten eine Abmischung mehrerer Substanzen ist. Vorzugsweise umfasst ein Analysedatensatz jedoch mehr der oben genannten Paare von Daten, bevorzugt mindestens 4, weiter bevorzugt mindestens 6 weiter bevorzugt mindestens 8 und meist bevorzugt mindestens 10 der oben genannten Paare von Daten.

Vorzugsweise umfasst ein Analysedatensatz Paare von Daten, die für Beschichtungszusammensetzungskomponenten und deren Anteilsgrößen charakteristisch sind, die in einem so großen Anteil in der Beschichtungszusammensetzung enthalten sind, dass sie einen signifikanten Einfluss auf mindestens eine Eigenschaft der Beschichtungszusammensetzung, insbesondere auf eine oder die eine Analysegröße haben. Da ein großer Anteil der Beschichtungszusammensetzung durch ein Lösungsmittel gebildet sein kann, kann der Anteil einer (oder mehrerer) anderen Beschichtungszusammensetzungskomponente(n) sehr klein sein, und die jeweilige Beschichtungszusammensetzungskomponente kann dennoch einen merklichen Einfluss auf die gesamte Beschichtungszusammensetzung haben. Dementsprechend ist vorzugsweise keine Untere Grenze der Anteilsgröße definiert. Es hat sich jedoch gezeigt, dass lediglich als Verunreinigung anzusehende Anteilsgrößen vernachlässigbar sein können. Vorzugsweise umfasst ein Analysedatensatz daher Paare von Daten, bei denen die jeweilige Anteilsgröße einen (Gewichts-) Anteil von mindestens 1:1.000.000 (bezogen auf das Gesamtgewicht aller Beschichtungszusammensetzungskomponenten in einer Beschichtungszusammensetzung, bevorzugt von mindestens 1:100.000, meist bevorzugt 1:10.000 aufweist.

Durch eine solche Beschränkung auf für mindestens eine Analysegröße relevante Paare von Daten in einem Analysedatensatz kann die Datenmenge vergleichsweise gering gehalten werden. So ist beispielsweise eine Beschränkung auf $\leq$ 50, bevorzugt $\leq$ 40, weiter bevorzugt $\leq$ 30, mehr bevorzugt $\leq$ 25 und meist bevorzugt $\leq$ 20 Paaren von Daten pro Analysedatensatz möglich.

Ebenso ist es vorteilhaft, die Anzahl der möglichen Beschichtungszusammensetzungskomponenten zu beschränken. Dies ist nicht nur im Hinblick auf die Größe der zu handhabenden Datenmenge vorteilhaft, sondern reduziert insbesondere auch die notwendige Rechenleistung. Vorteilhaft ist daher die Anzahl erlaubter Beschichtungszusammensetzungskomponenten $\leq$ 1000, bevorzugt $\leq$ 750, weiter bevorzugt $\leq$ 500, mehr bevorzugt $\leq$ 400 und meist bevorzugt $\leq$ 350. Dabei werden vorzugsweise diejenigen Beschichtungszusammensetzungskomponenten ausgewählt, die in Marktbekannten Beschichtungszusammensetzungen enthalten sind und/oder von denen man weiß oder zumindest vermutet, dass sie einen signifikanten Einfluss auf mindestens eine Analysegröße haben.

Bevorzugt umfasst jeder Analysedatensatz nicht nur eine einzige Analysegröße. Es ist bei Beschichtungszusammensetzungen üblich, diese durch mehrere (für Anwender relevante) Daten zu charakterisieren. Diese Daten werden für eine Beschichtungszusammensetzung daher meist ohnehin ermittelt, so dass sie zur Verfügung stehen und vergleichsweise einfach in einen Analysedatensatz eingefügt werden können. Die Angabe mehrerer Analysegrößen in einem Analysedatensatz ist vorteilhaft, da so bei der Ermittlung der physiko-chemischen Beschichtungszusammensetzungseigenschaft nicht nur diese, sondern auch andere Beschichtungszusammensetzungseigenschaften, insbesondere diejenigen, die der Analysegröße entsprechen, ermittelbar sind. Ein Fachmann (oder ein Computersystem) kann somit unmittelbar eine Plausibilitätsprüfung durchführen und Ermittlungsresultate ausschließen oder verwerfen, für die mindestens eine Beschichtungszusammensetzungseigenschaft beziehungsweise eine Analysegröße außerhalb eines zulässigen Bereichs liegt. Bevorzugt umfasst daher ein Analysedatensatz $\geq$ 5 Analysegrößen, bevorzugt $\geq$ 8, weiter bevorzugt $\geq$ 12, mehr bevorzugt $\geq$ 15, insbesondere bevorzugt $\geq$ 20, meist bevorzugt $\geq$ 25 Analysegrößen.

Es hat sich gezeigt, dass eine Datenbank eine Mindestanzahl von Analysedatensätzen umfassen sollte, um eine zuverlässige Ermittlung einer Beschichtungszusammensetzungseigenschaft zu ermöglichen. Daher sollte eine Datenbank $\geq$ 50, bevorzugt $\geq$ 100, weiter bevorzugt $\geq$ 150, mehr bevorzugt $\geq$ 175, meist bevorzugt $\geq$ 200 Analysedatensätze umfassen. Ermittlungen einer Beschichtungszusammensetzungseigenschaft können mit einer Datenbank dieser Größe mit akzeptabler Fehlertoleranz ermittelt werden. Denkbar und in vielen Fällen bevorzugt sind jedoch größere Datenbanken mit beispielsweise $\geq$ 500, bevorzugt $\geq$ 1000, weiter bevorzugt $\geq$ 1500, mehr bevorzugt $\geq$ 2000, meist bevorzugt $\geq$ 5000 Analysedatensätzen. Auch wenn bei diesen Datenbanken die zu handhabende Datenmenge ansteigt, hat sich dies in einigen Fällen als vorteilhaft erwiesen, da auf Basis einer derartigen Datenbank die Beschichtungszusammensetzungseigenschaft mit höherer Genauigkeit und Robustheit ermittelbar ist.

Weiterhin ist für das erfindungsgemäße Verfahren wesentlich, das Computer-implementierte Ermitteln von wenigstens einer für die Beschichtungszusammensetzungseigenschaft der ersten Beschichtungszusammensetzung charakteristischen physiko-chemischen Eigenschaftsgröße auf Grundlage des Analysedatensatzes zu der zweiten Beschichtungszusammensetzung und des mindestens einen weiteren Analysedatensatzes zu der mindestens einen dritten Beschichtungszusammensetzung.

Vorzugsweise werden mehr als zwei Analysedatensätze, beispielsweise ≥ 5 Analysedatensätze vorzugsweise ≥ 10 Analysedatensätze, weiter bevorzugt ≥ 20 Analysedatensätze und meist bevorzugt ≥ 50 Analysedatensätze herangezogen, um die wenigstens eine für die Beschichtungszusammensetzungseigenschaft der ersten Beschichtungszusammensetzung charakteristische physiko-chemischen Eigenschaftsgröße zu ermitteln.

Vorzugsweise umfasst der erste und/oder der zweite Analysedatensatz (insbesondere bevorzugt jeder Analysedatensatz) wenigstens eine Analysegröße und bevorzugt eine Vielzahl von Analysegrößen, beispielsweise ≥ 5 Analysegrößen, vorzugsweise ≥ 8 Analysegrößen, weiter bevorzugt ≥ 10 Analysegrößen und meist bevorzugt ≥ 15 Analysegrößen. Bevorzugt ist jede der Analysegrößen für eine Beschichtungszusammensetzungseigenschaft charakteristisch. Eine Analysegröße kann mit anderen Worten als Identifikator (nachfolgend auch als "Identifier" bezeichnet) einer Eigenschaft einer Beschichtungszusammensetzung dienen, welche insbesondere betrachtet werden soll.

Vorzugsweise ist die Beschichtungszusammensetzungseigenschaft, die wie oben dargelegt bevorzugt durch die Analysegröße charakterisiert ist, ausgewählt aus einer Gruppe, die einen Abrasionswiderstand, eine Nassabriebbeständigkeit, ein Deckvermögen, einen Farbwert, einen Gelbwert, ein Weißgrad, einen Glanz, eine Dichte, eine Auftragsmenge, eine Ausbeute, eine Ergiebigkeit, eine Viskosität, einen Feststoffanteil, einen Lösungsmittelanteil, eine Wasserlöslichkeit, eine Wasseremulgierbarkeit, eine Wassersuspendierbarkeit, eine UV-Beständigkeit, eine Temperaturwiderstandfähigkeit, eine Lagerfähigkeit, eine Diffusionseigenschaft, eine Atmungsaktivät, einen Widerstandsfähigkeit, einen pH-Wert, einen Schadstoffwert, eine Hitzewiderstandsfähigkeit, eine Feuerfestigkeitsfähigkeit, eine angemessene Entsorgungsmöglichkeit für die Beschichtungszusammensetzung und ein Gesundheitsgefährdungsrisiko umfasst. Diese Beschichtungszusammensetzungseigenschaften können dazu dienen, Beschichtungszusammensetzungen voneinander zu unterscheiden und deren Eignung für bestimmte Anwendungsfälle zu bewerten.

Vorzugsweise enthält ein Analysedatensatz (insbesondere bevorzugt jeder Analysedatensatz) mindestens eine Anteilsgröße, welche für einen Anteil einer Beschichtungszusammensetzungskomponente an der jeweiligen Beschichtungszusammensetzung charakteristisch ist. Vorzugsweise enthält ein Analysedatensatz (vorzugsweise mehrere Analysedatensätze, meist bevorzugt jeder Analysedatensatz) für (zumindest nahezu) jede (oder jede bewusst zugesetzte) in der Beschichtungszusammensetzung enthaltene Beschichtungszusammensetzungskomponente eine Anteilsgröße, welche für den Anteil dieser Beschichtungszusammensetzungskomponente an der jeweiligen Beschichtungszusammensetzung charakteristisch ist.

So könnte beispielsweise ein Analysedatensatz, welcher eine rote Beschichtungszusammensetzung charakterisiert zumindest eine Anteilsgröße enthalten, welche für einen Anteil eines roten Farbpigments charakteristisch ist. Daneben könnte dieser Analysedatensatz beispielsweise auch eine oder mehrere weitere Anteilsgröße/n umfassen, welche/r beispielsweise für einen Anteil eines Lösungsmittels, eines Bindemittels und/oder eines Biozids charakteristisch ist/sind. Eine Analysegröße könnte in einem solchen beispielhaften Analysedatensatz beispielsweise ein Deckvermögen, ein Farbwert und/oder eine Wellenlänge (oder mehrere Wellenlängen oder sogar ein Wellenlängenspektrum) sein.

Vorzugsweise handelt es sich bei der ersten Beschichtungszusammensetzungskomponente und der wenigstens einen weiteren Beschichtungszusammensetzungskomponente jeweils um einen Inhaltsstoff der Beschichtungszusammensetzung, vorzugsweise um ein Additiv. Ein Lösungsmittel kann jedoch auch als eine erste Beschichtungszusammensetzungskomponente oder ein der wenigstens einen weiteren Beschichtungszusammensetzungskomponente betrachtet werden.

Bevorzugt ist die erste Beschichtungszusammensetzungskomponente und/oder wenigstens eine weitere Beschichtungszusammensetzungskomponente ausgewählt ist aus einer Gruppe, die Füllstoff, Rheologieadditiv, Pigment, Farbstoff, Emulgator, Film- und Topfkonservierungsmittel umfasst.

Insbesondere ist bevorzugt, dass die erste Beschichtungszusammensetzungskomponente und/oder wenigstens eine weitere Beschichtungszusammensetzungskomponente ein Kaolin- und/oder Kaolinfolgeprodukt und/oder eine Ca- und/oder Mg-haltige Komponente ist.

Bevorzugt ist außerdem oder ergänzend zu oben beschriebenen Ausführungsformen, dass in einem Analysedatensatz jeder Beschichtungszusammensetzungskomponente (insbesondere jeweils genau) eine Anteilsgröße zugeordnet ist. Vorzugsweise bezieht sich folglich jede Anteilsgröße auf eine ihr zugeordnete (vorgegebene und/oder vorgebbare) Beschichtungszusammensetzungskomponente der jeweiligen Beschichtungszusammensetzung, welche durch den jeweiligen Analysedatensatz charakterisiert ist.

Vorzugsweise wird der erste Analysedatensatz durch eine Analyse, vorzugsweise eine Vollanalyse, der zweiten Beschichtungszusammensetzung erhalten wird und/oder der zweite Analysedatensatz durch eine Analyse, vorzugsweise eine Vollanalyse, der weiteren Beschichtungszusammensetzung (erhalten. Eine Analyse kann dabei darauf beschränkt sein, einen oder mehrere der in dem Analysedatensatz enthaltenen Daten zu ermitteln. Alternativ zu einer Analyse aller Daten des Datensatzes ein Datum oder mehrere Daten, insbesondere eine Anteilsgröße oder mehrere Anteilsgrößen, auch einer Rezeptur oder Formulierungsanweisung entnommen oder daraus berechnet werden. Insbesondere ist bevorzugt, dass eine Analysegröße oder mehre Analysegrößen durch eine Analyse der jeweiligen Eigenschaft der Beschichtungszusammensetzung ermittelt wird/werden und/oder die Anteilsgrößen der ersten Beschichtungszusammensetzungskomponente und/oder der weiteren Beschichtungszusammensetzungskomponenten einer Rezeptur oder Formulierungsanweisung entnommen oder daraus berechnet werden.

Bevorzugt erfolgt die Ermittlung der wenigstens einen physiko-chemischen Beschichtungszusammensetzungseigenschaft der ersten Beschichtungszusammensetzung auf Grundlage von zu einer Vielzahl verschiedener Beschichtungszusammensetzungen jeweils erfassten Analysedatensätzen. Bei dieser Verfahrensvariante ist es somit möglich, eine physiko-chemischen Beschichtungszusammensetzungseigenschaft zu ermitteln, ohne die erste Beschichtungszusammensetzung tatsächlich auf diese Beschichtungszusammensetzungseigenschaft hin tatsächlich physiko-chemisch analysieren zu müssen. Vielmehr ist es möglich, diese physiko-chemischen Beschichtungszusammensetzungseigenschaft (vorzugsweise prädiktiv) rechnerisch (vorzugsweise computergestützt) zu ermitteln. Als Basis für diese Berechnungen werden die Daten von Analysedatensätzen herangezogen, die beispielsweise in einer Datenbank für diesen Zweck vorgehalten werden.

Bevorzugt ist jeder Analysedatensatz (bevorzugt durch die Werte der Anteilsgrößen, welche den jeweiligen Beschichtungszusammensetzungskomponenten zugeordnet sind) jeweils charakteristisch für eine Beschichtungszusammensetzung, so dass insbesondere bevorzugt die verschiedenen Beschichtungszusammensetzungen auf Grundlage der jeweiligen Analysedatensätze voneinander (bevorzugt eineindeutig) unterscheidbar sind. Denkbar wäre aber auch, dass zwei (oder mehr) Beschichtungszusammensetzungen derart ähnlich in ihrer Beschichtungszusammensetzungen sind, dass ihre jeweiligen Analysedatensätze (im Wesentlichen bzw. ggf. im Rahmen einer (Mess-)Toleranz) gleich sind.

In einer bevorzugten Ausführungsform kann wenigstens eine Beschichtungszusammensetzung und bevorzugt kann jede Beschichtungszusammensetzung verknüpft sein mit einer Beschichtungszusammensetzungsidentifikationsgröße (einer sogenannten "Beschichtungszusammensetzungs-ID"), welche charakteristisch ist für die jeweilige Beschichtungszusammensetzung und/oder einer Charge der Beschichtungszusammensetzung und/oder eines Erzeugers der Beschichtungszusammensetzung und/oder einer Lagermodalität und/oder einer Transportmodalität der Beschichtungszusammensetzung (etwa ein Transportmittel und/oder ein Transportbehältnis und/oder eine Vorfüllung des Transportbehältnisses und/oder dergleichen).

Bevorzugt wird der Analysedatensatz wenigstens einer Beschichtungszusammensetzung in Abhängigkeit der der jeweiligen Beschichtungszusammensetzung zugeordneten Beschichtungszusammensetzungsidentifikationsgröße (insbesondere eine sogenannte Beschichtungszusammensetzungs-ID) erfasst, etwa durch Abrufen von einer Datenbank (welche beispielsweise mittels einer externen Speichereinrichtung und/oder einem externen Server bereitgestellt sein kann), in welcher bevorzugt eine Vielzahl von Paaren aus einer Beschichtungszusammensetzungsidentifikationsgröße und dieser jeweils zugeordnetem Analysedatensatz oder hierfür charakteristische Daten abgelegt ist.

Bevorzugt ist die Beschichtungszusammensetzungsidentifikationsgröße einzigartig und/oder eindeutig und besonders bevorzugt eineindeutig für die jeweilige Beschichtungszusammensetzung. Denkbar wäre in diesem Zusammenhang beispielsweise, dass ein erster Analysedatensatz einer Beschichtungszusammensetzung, welche unter bestimmten Bedingungen für eine vorgegebene Zeit gelagert wurde eine erste Beschichtungszusammensetzungsidentifikationsgröße zugewiesen bekommt, wohingegen einer ursprünglich identische Beschichtungszusammensetzung, welche unter anderen Bedingungen und/oder über eine andere Zeit gelagert wurde eine andere Beschichtungszusammensetzungs-ID zugewiesen wird.

Dabei ist zu beachten, dass sich die Analysedatensätze zweier ursprünglich identischer Beschichtungszusammensetzungen unterscheiden können, da aufgrund der unterschiedlichen Lagerung und/oder Transportmodalität eine Analysegröße oder mehrere der Analysegrößen verändert sein könnten.

Das oben beschriebene Verfahren kann auch dazu genutzt werden, Eigenschaften von Mischungen verschiedener Beschichtungszusammensetzungen vorherzusagen. Sollen beispielsweise zwei oder mehrere Beschichtungszusammensetzungen bekannter bzw. ermittelbarer Analysegrößen (bevorzugt mit bekannten Beschichtungszusammensetzungs-IDs) vermischt werden (beispielsweise um einen anderen Farbton zu erzeugen), kann zur Ermittlung der Beschichtungszusammensetzungseigenschaft des daraus entstehenden Beschichtungszusammensetzungsgemischs der jeweilige Analysedatensatz oder hierfür charakteristische Daten von einer derartigen Datenbank beispielsweise auf Grundlage der bekannten bzw. ermittelbaren Beschichtungszusammensetzungsidentifikationsgrößen abgerufen werden (und hierdurch erfasst werden).

Weiterhin ist eine Verfahrensvariante bevorzugt, bei der ergänzend oder alternativ zu einer oder mehreren der oben dargelegten Varianten auf Grundlage eines Soll-Wertes wenigstens einer und bevorzugt einer Vielzahl von Beschichtungszusammensetzungseigenschaften beziehungsweise Beschichtungszusammensetzungseigenschaftsgrößen der Beschichtungszusammensetzung eine der Beschichtungszusammensetzung zuzuführende erste Beschichtungszusammensetzungskomponente und deren Anteilsgröße ermittelt wird und/oder eine der Beschichtungszusammensetzung zuzuführende weitere Beschichtungszusammensetzungskomponente und deren jeweilige Anteilsgröße ermittelt wird. Wird also beispielsweise eine bestimmte Beschichtungszusammensetzungseigenschaft, beispielsweise ein Farbwert, vorgegeben, kann durch diese Verfahrensvariante ermittelt werden, welche erste Beschichtungszusammensetzungskomponente und/oder weitere Beschichtungszusammensetzungskomponente einer Beschichtungszusammensetzung in welcher jeweiliger Anteilsgröße zugeführt werden sollte, um zu gewährleisten, dass die Beschichtungszusammensetzung tatsächlich den Soll-Wert, nämlich den vorgegebenen Farbwert aufweist.

In einer bevorzugten Verfahrensvariante erfolgt die computer-implementierte Ermittlung von wenigstens einer für die Beschichtungszusammensetzungseigenschaft der ersten Beschichtungszusammensetzung charakteristischen physiko-chemischen Eigenschaftsgröße rein computer-implementiert. Dazu wird wie oben beschrieben vorzugsweise der Analysedatensatz zu der zweiten Beschichtungszusammensetzung und der mindestens eine weitere Analysedatensatz zu der mindestens einen dritten Beschichtungszusammensetzung zugrunde gelegt.

Bevorzugt erfolgt somit das Ermitteln der wenigstens einen Eigenschaftsgröße in einem Computer-implementierten Verfahrensschritt. Bevorzugt erfolgt das Ermitteln der wenigstens einen Eigenschaftsgröße mittels einer bzw. der (insbesondere nachfolgend beschriebenen), insbesondere Prozessor-basierten, Beschichtungszusammensetzungseigenschafts-Ermittlungsvorrichtung.

Eine Ermittlung der Beschichtungszusammensetzungseigenschaft auf Daten, die bereits bei der Produktion der Beschichtungszusammensetzung bekannt sind (nämlich insbesondere die eingesetzten Beschichtungszusammensetzungskomponenten und deren jeweilige Anteilsgrößen), und die gegebenenfalls im Verlauf des Transports und Lagerns erneut überprüft werden, ist dabei mit deutlich weniger Aufwand verbunden (im Vergleich zu einer experimentellen Messung der Beschichtungszusammensetzungseigenschaften, sowie der Analyse der Beschichtungszusammensetzungskomponenten deren jeweiligen Anteilsgrößen einer zuvor unbekannten Beschichtungszusammensetzung).

Bevorzugt wird zur Ermittlung der wenigstens einen Beschichtungszusammensetzungseigenschaft beziehungsweise der Beschichtungszusammensetzungseigenschaftsgröße ein Analysedatensatz zu der ersten Beschichtungszusammensetzung ermittelt bzw. berechnet. Die Ermittlung des Analysedatensatzes zu der Beschichtungszusammensetzung erfolgt bevorzugt unter Berücksichtigung des ersten Analysedatensatzes zu einer zweiten Beschichtungszusammensetzung und mindestens eines weiteren Analysedatensatzes zu mindestens einer dritten Beschichtungszusammensetzung.

Bevorzugt wird der Analysedatensatz der ersten Beschichtungszusammensetzung derart ermittelt, dass zu der wenigstens einen physiko-chemischen Beschichtungszusammensetzungseigenschaft, im Folgenden auch als Soll-Eigenschaft bezeichnet, und bevorzugt zu jeder (vorgegebenen) Beschichtungszusammensetzungseigenschaft eine erste Beschichtungszusammensetzungskomponente sowie deren Anteilsgröße und wenigstens eine weitere, von der ersten Beschichtungszusammensetzungskomponente verschiedene Beschichtungszusammensetzungskomponente verschiedene Beschichtungszusammensetzungskomponente und deren Anteilsgröße ermittelt wird. Die Ermittlung der ersten Beschichtungszusammensetzungskomponente sowie deren Anteilsgröße als auch der wenigstens einen weiteren Beschichtungszusammensetzungskomponente und deren Anteilsgröße erfolgt vorzugsweise auf Grundlage eines ersten Analysedatensatzes zu einer zweiten Beschichtungszusammensetzung und mindestens eines weiteren Analysedatensatzes zu mindestens einer dritten Beschichtungszusammensetzung.

Bevorzugt wird für die physiko-chemischen Beschichtungszusammensetzungseigenschaft einer ersten Beschichtungszusammensetzung (die Soll-Eigenschaft beziehungsweise eine dazu korrelierende Analysegröße) basierend auf einem Einfluss der Anteilsgröße der ersten Beschichtungszusammensetzungskomponente sowie einem Einfluss der Anteilsgröße, der wenigstens einen weiteren Beschichtungszusammensetzungskomponente auf die Soll-Eigenschaft bzw. die Analysegröße berechnet. Bevorzugt wird dazu einer, bevorzugt mehreren, meist bevorzugt jeder Beschichtungszusammensetzungskomponente ein Gewichtungswert zugeordnet, welcher zu einem Einfluss der jeweiligen Anteilsgröße der wenigstens einen weiteren Beschichtungszusammensetzungskomponente auf eine Veränderung der Soll-Eigenschaft korreliert. So könnte beispielsweise einem Rotpigment als Beschichtungszusammensetzungskomponente ein hoher Gewichtungswert für die Soll-Eigenschaft eines roten Farbtons der ersten Beschichtungszusammensetzung zugeordnet werden, jedoch ein niedriger Gewichtungswert für die Soll-Eigenschaft einer Viskosität der ersten Beschichtungszusammensetzung. Einem Lösungsmittel als Beschichtungszusammensetzungskomponente hingegen könnte beispielsweise ein niedriger Gewichtungswert für die Soll-Eigenschaft einer bestimmten Farbe der ersten Beschichtungszusammensetzung zugeordnet werden, jedoch ein hoher Gewichtungswert für die Soll-Eigenschaft der Viskosität der ersten Beschichtungszusammensetzung.

Mit anderen Worten wird vorzugsweise für jede (vorgegebene) Beschichtungszusammensetzungseigenschaft einer ersten Beschichtungszusammensetzung separat die eine Anteilsgröße sowohl der ersten Beschichtungszusammensetzungskomponente als auch der wenigstens einen weitere, von der ersten Beschichtungszusammensetzungskomponente verschiedenen Beschichtungszusammensetzungskomponente bestimmt bzw. berechnet.

Bevorzugt umfasst mindestens der erste und der zweite Analysedatensatz, vorzugsweise eine Vielzahl der zur Ermittlung der physiko-chemischen Beschichtungszusammensetzungseigenschaft der ersten Beschichtungszusammensetzung zur Verfügung stehenden Analysedatensätze, einen Wert der für diese physiko-chemischen Beschichtungszusammensetzungseigenschaft (jedoch nicht der ersten sondern der durch den jeweiligen Analysedatensatz charakterisierten zweiten oder weiteren Beschichtungszusammensetzung) charakteristisch ist. Dadurch wird ermöglicht, dass jeder der Beschichtungszusammensetzungskomponenten der zweiten oder weiteren Beschichtungszusammensetzung jeweils ein Gewichtungswert zugeordnet werden kann, welcher den Einfluss der jeweiligen Beschichtungszusammensetzungskomponente auf die Beschichtungszusammensetzungseigenschaft abbildet.

Die Verwendung derartiger Analysedatensätze und eine Ermittlung von Gewichtungswerten für die jeweiligen Beschichtungszusammensetzungskomponenten, bietet den Vorteil, dass die (hierfür) zu übermittelnde Datenmenge nicht so groß (wie im Stand der Technik bzw. bei Vollanalysen) ist und (wie nachfolgend erläutert) eine viel kleinere Anzahl an Datensätzen erforderlich ist, um ein robustes Ergebnis zu erhalten. Die Berechnung bzw. Ermittlung der physiko-chemischen Beschichtungszusammensetzungseigenschaft ist sehr schnell möglich und erfordert keine großen Versuchsreihen mit einer Vielzahl von verschiedenen Formulierungen zur Bereitstellung von Beschichtungszusammensetzungen für eine anschließende (physiko-chemische) Analyse.

Das vorgeschlagene Verfahren, insbesondere die Verwendung eines reduzierten Datensatzes (durch Verwendung der jeweiligen Analysedatensätze der Beschichtungszusammensetzungen), bietet den Vorteil, dass es keine bzw. stark verringerte unbekannte Bereiche aufweist. Diese Daten (der Analysedatensätze) werden vorzugsweise standardmäßig bei der Herstellung der jeweiligen Beschichtungszusammensetzung bereitgestellt. Die Menge an zu übermittelnden Daten ist gegenüber den Einzelkomponenten erheblich kleiner. Durch die bevorzugte Verwendung von funktional aggregierten Gruppen wird ein robusteres Ergebnis erzeugt.

Bevorzugt weist der (insbesondere jeder) Analysedatensatz vorzugsweise ≤ 50, bevorzugt ≤ 30, weiter bevorzugt ≤ 25, meist bevorzugt ≤ 20 verschiedene Analysegrößen. Grundsätzlich wäre es auch möglich, Analysedatensätze mit einer größeren Anzahl verschiedener Analysegrößen zu nutzen, jedoch wird sich im Hinblick auf die Menge der zu speichernden Daten sowie der geforderten Rechenleistung vorzugsweise auf die oben genannte Anzahl der relevantesten Analysegrößen beschränkt. Für die meisten kommerziell erhältlichen Beschichtungszusammensetzungen wird auch lediglich eine Anzahl relevanter Kenndaten zur Charakterisierung der Produkteigenschaften in dem oben genannten Bereich angegeben.

Als untere Grenze weist der (insbesondere jeder) Analysedatensatz bevorzugt mindestens eine Analysegröße auf. So wäre beispielsweise denkbar, dass ausschließlich die Farbe der Beschichtungszusammensetzungen als einzige Analysegröße verwendet würde. Diese Information könnte beispielsweise zum Abmischen verschiedener Beschichtungszusammensetzungen zur Erzeugung eines neuen Farbtons herangezogen werden. Bevorzugt ist jedoch, dass in einem (insbesondere jedem) Analysedatensatz mehrere Analysegrößen gespeichert sind. Bevorzugt weist der (insbesondere jeder) Analysedatensatz mindestens 3, bevorzugt ≥ 5, weiter bevorzugt ≥ 8, mehr bevorzugt ≥ 10 und meist bevorzugt ≥ 15 verschiedene Analysegrößen auf.

Die vorgenannten maximalen Anzahlen für die jeweiligen Analysegrößen bieten jeweils (einzeln oder in Kombination) den Vorteil, dass zum einen typische Daten, welche bei der Herstellung der Beschichtungszusammensetzungen üblicherweise bestimmt werden, verwendet werden können und keine weiteren Messdaten zur Erfassung des Analysedatensatzes erzeugt bzw. bestimmt werden müssen und gleichzeitig eine möglichst weitgehende Reduktion der Datensätze der Analysedatensätze vorgenommen werden kann.

Bei einem weiter bevorzugten Verfahren erfolgt die Ermittlung der wenigstens einen Beschichtungszusammensetzungseigenschaft bzw. einer dazu korrelierenden Eigenschaftsgröße auf Grundlage von zu einer Vielzahl verschiedener (Trainings-) Beschichtungszusammensetzungen jeweils erfassten Analysedatensätzen, welche insbesondere Informationen zu Beschichtungszusammensetzungskomponenten sowie deren jeweilige Anteilsgröße in einer Beschichtungszusammensetzung umfassen. Ein Analysedatensatz ist damit vorzugsweise geeignet, eine Beschichtungszusammensetzung hinsichtlich mindestens einer Beschichtungszusammensetzungseigenschaft und mindestens zwei Beschichtungszusammensetzungskomponenten sowie deren jeweilige Anteilsgröße zu charakterisieren.

Der Ausdruck "Trainings-Beschichtungszusammensetzung" ist insbesondere so zu verstehen, dass sich diese Trainings-Beschichtungszusammensetzung von der Beschichtungszusammensetzung unterscheidet, von der die wenigstens eine physiko-chemischen Beschichtungszusammensetzungseigenschaft ermittelt werden soll. Vorzugsweise eine Vielzahl derartiger "Trainings-Beschichtungszusammensetzungen" werden insbesondere im zeitlichen Vorlauf zur Ermittlung der Beschichtungszusammensetzungseigenschaft der (ersten) Beschichtungszusammensetzung ausgewertet (und beispielsweise analysiert und/oder Rezepturdaten herangezogen) und auf Grundlage deren Auswertung kann auf eine Beschichtungszusammensetzungseigenschaft der Beschichtungszusammensetzung geschlossen werden. Insbesondere handelt es sich bei den Trainings-Beschichtungszusammensetzungen insofern um bekannte Beschichtungszusammensetzungen, als zu diesen Messdaten bzw. Messgröße(n) oder hiervon abgeleitete Eigenschaftsgröße(n) vorliegen, welche für die wenigstens eine Analysegröße charakteristisch sind.

Das vorgeschlagene Verfahren bietet im Vergleich zu dem aus dem eingangs beschriebenen Stand der Technik bekannten Verfahren den Vorteil verringerter Kosten bei der Bereitstellung der Daten. Weiter vorteilhaft kann ein Zurückgreifen bei der Datenbasis auf einen etablierten und angewandten Standard erfolgen. Durch das vorgeschlagene Verfahren kann ein robustes Ergebnis erreicht werden und Parameter (insbesondere für die Beschichtungszusammensetzungseigenschaft) ermittelt werden, die sonst nicht rechnerisch vorhersagebar wären bzw. mittels einer auf Einzelkomponenten basierenden Ermittlung fehleranfälliger und/oder mit größeren Abweichen verhaftet wären.

Bevorzugt wird auf Grundlage der Vielzahl von der zu den Trainings-Beschichtungszusammensetzungen ermittelten bzw. erfassten Analysedatensätzen sowie auf Grundlage der diesen zugeordneten Analysegrößen, welche charakteristisch sind für wenigstens eine Beschichtungszusammensetzungseigenschaft, ein insbesondere auf einem (künstlichen) neuronalen Netz basierenden und/ oder eines Beschichtungszusammensetzungseigenschafts-Ermittlungsmodell maschinellen Lernens zur Ermittlung wenigstens einer Beschichtungszusammensetzungseigenschaft trainiert. Dieses Beschichtungszusammensetzungseigenschafts-Ermittlungsmodell wird nachfolgend näher beschrieben. Bevorzugt wird dabei ein nachfolgend näher beschriebenes Beschichtungszusammensetzungseigenschafts-Ermittlungsmodell zur Ermittlung wenigstens einer (physiko-chemischen) Beschichtungszusammensetzungseigenschaft (einer ersten Beschichtungszusammensetzung) gemäß einer bevorzugten Ausführungsform zur Ermittlung der wenigstens einen Eigenschaftsgröße verwendet.

Bevorzugt bezieht sich die vorliegende Erfindung auf ein Al-ML-Verfahren zur Bestimmung von mindestens einer Beschichtungszusammensetzungseigenschaft.

Bei einem weiter bevorzugten Verfahren erfolgt die Ermittlung der wenigstens einen physiko-chemischen Beschichtungszusammensetzungseigenschaft (beispielsweise mittels einer Beschichtungszusammensetzungseigenschafts-Ermittlungsvorrichtung) in Abhängigkeit eines, insbesondere trainierbaren, Beschichtungszusammensetzungseigenschafts-Ermittlungsmodells maschinellen Lernens, welches einen Satz insbesondere trainierbarer, Parameter umfasst, welche auf Werte eingestellt sind, die als Ergebnis eines Trainingsprozesses gelernt wurden, wobei der Trainingsprozess auf Grundlage eines Satzes von Trainingsdaten erfolgt ist.

Bevorzugt umfassen die Trainingsdaten wenigstens einen ersten Analysedatensatz zu einer zweiten Beschichtungszusammensetzung, wobei der erste Analysedatensatz wenigstens eine Analysegröße und eine Anteilsgröße einer ersten Beschichtungszusammensetzungskomponente und bevorzugt einer Vielzahl von Anteilsgrößen von weiteren Beschichtungszusammensetzungskomponenten umfasst, sowie mindestens eines weiteren Analysedatensatz zu mindestens einer dritten Beschichtungszusammensetzung, wobei der zweite Analysedatensatz wenigstens eine Analysegröße und eine Anteilsgröße einer ersten Beschichtungszusammensetzungskomponente und bevorzugt einer Vielzahl von Anteilsgrößen von weiteren Beschichtungszusammensetzungskomponenten enthält. Bei den Beschichtungszusammensetzungskomponenten und deren Analysegrößen handelt es sich insbesondere bevorzugt, um Daten, die eine zur Ermittlung der Beschichtungszusammensetzungseigenschaft charakteristische Größe umfassen.

Bevorzugt basiert das Beschichtungszusammensetzungseigenschafts-Ermittlungsmodell maschinellen Lernens auf einem (künstlichen) neuronalen Netzwerk. Bevorzugt ist das neuronale Netzwerk als tiefes neuronales Netzwerk (Deep Neural Network, DNN), bei dem die parametrierbare Verarbeitungskette eine Mehrzahl von Verarbeitungsschichten aufweist, und/oder ein sogenanntes Convolutional Neural Network (CNN) (dt. Faltungsnetzwerk) und/oder ein rekurrentes Neuronales Netzwerk (RNN, engl. Recurrent Neural Network) ausgebildet.

Bevorzugt werden dem Beschichtungszusammensetzungseigenschafts-Ermittlungsmodell bzw. dem (künstlichen) neuronalen Netzwerk die (zu verarbeitenden) Daten, insbesondere der oben genannten erste Analysedatensatz und der mindestens eine weitere Analysedatensatz (oder hiervon abgeleitete Daten) als Eingangsgrößen zugeführt. Bevorzugt bildet das Beschichtungszusammensetzungseigenschafts-Ermittlungsmodell bzw. das künstliche neuronale Netzwerk die Eingangsgrößen in Abhängigkeit einer parametrierbaren Verarbeitungskette auf Ausgangsgrößen ab.

Bevorzugt ist als Ausgangsgröße wenigstens eine Größe gewählt, die für eine Beschichtungszusammensetzungseigenschaft (beispielsweise einen Farbwert, einen Viskositätswert, ein Deckvermögen) charakteristisch ist.

Es ist jedoch auch denkbar, , einen Soll-Wert der physiko-chemischen Beschichtungszusammensetzungseigenschaft vorzugeben und das Verfahren zu nutzen, um Deskriptoren für eine Beschichtungszusammensetzung zu ermitteln, welche (gemäß dieser Ermittlung wahrscheinlich) die vorgegebene Beschichtungszusammensetzungseigenschaft aufweist. Bei dieser Variante des Verfahrens wird somit ein Soll-Wert der physiko-chemischen Beschichtungszusammensetzungseigenschaft vorgegeben und die Beschichtungszusammensetzungskomponenten sowie die diesen Beschichtungszusammensetzungskomponenten zugehörigen Anteilsgrößen ermittelt. Diese Verfahrensvariante ist besonders dann vorteilhaft, wenn nach Formulierungen für eine Beschichtungszusammensetzung gesucht wird, die eine bestimmte Soll-Eigenschaft aufweisen soll.

Bevorzugt wird/wurde das Beschichtungszusammensetzungseigenschafts-Ermittlungsmodell maschinellen Lernens unter Verwendung vorgegebener Trainingsdaten trainiert, wobei durch das Training die parametrierbare Verarbeitungskette parametriert wird.

Bei einem bevorzugten Verfahren werden in dem Trainingsprozess des Beschichtungszusammensetzungseigenschafts-Ermittlungsmodells Trainingsdaten verwendet, welche zumindest eine Analysegröße und/oder für Beschichtungszusammensetzungskomponenten charakteristische Größen sowie die diesen Beschichtungszusammensetzungskomponenten zugeordneten Anteilsgrößen umfassen. Diese Daten können im Rahmen einer Analyse ermittelt sein (bevorzugt für die Analysegröße) und/oder im Rahmen der Herstellung der Beschichtungszusammensetzungen, auf denen die Trainingsdaten beruhen (beispielsweise aus der Rezeptur oder Überwachungseinrichtungen der genutzten Gerätschaften (bevorzugt für die Beschichtungszusammensetzungskomponenten und deren Anteilsgrößen) umfassen. Denkbar ist auch, dass Trainingsdaten auf Grundlage von Beschichtungszusammensetzungen verschiedener Chargen (optional verschiedener Anbieter und/oder Rohstoffen verschiedener Quellen) verwendet werden. Dies bietet den Vorteil, dass in kurzer Zeit eine hohe Zahl an Trainingsdaten erzeugt werden können.

Bevorzugt werden die zur Verwendung als Trainingsdaten vorgesehenen ermittelten Analysegrößen mit weiteren Merkmalen wie beispielsweise einem Analysemerkmal (welches beispielsweise eine oder mehr Größen umfasst, die für die Art, Methode, Dienstleiser der/die für die Ermittlung dieser Analysegröße charakteristisch ist), einem Herstellermerkmal, einem Lagermerkmal (welches beispielsweise eine oder mehr Größen umfasst, die für die Art, Dauer, Temperatur, Lagerbehältnis oder eine andere lagerbedingte Eigenschaft charakteristisch ist) und/oder einem Transportmerkmal (welches beispielsweise eine oder mehr Größen umfasst, die für die Art, Dauer, Strecke, Temperatur, Transportbehältnis oder eine andere transportbedingte Eigenschaft charakteristisch ist) versehen.

Bevorzugt werden die erfassten Analysedatensätze, optional zusammen mit den jeweils ihnen zugeordneten weiteren Merkmalen (beispielsweise Analysemerkmal, Herstellermerkmal, Lagermerkmal und/oder Transportmerkmal als Trainingsdatensatz (insbesondere auf einer und/oder der nicht-flüchtigen Speichereinrichtung) abgelegt und/oder verwendet. Bevorzugt wird auf diese Weise eine Vielzahl von Trainings-Datensätzen bereitgestellt.

Durch die Variation der Anteilsgrößen einer oder mehrerer Beschichtungszusammensetzungskomponente/n und die Korrelation zu der oder den Analysegrößen ist es theoretisch möglich, eine Korrelation zwischen den Anteilsgrößen einer oder mehrerer Beschichtungszusammensetzungskomponente/n und der Analysegrößen beziehungsweise der Beschichtungszusammensetzungseigenschaft zu ermitteln.

Bevorzugt wird ein derart trainiertes neuronales Netzwerk (als Beschichtungszusammensetzungseigenschafts-Ermittlungsmodell) eingesetzt. Bevorzugt erfolgt das Training mittels überwachtem Lernen. Es wäre jedoch auch möglich, das Beschichtungszusammensetzungseigenschafts-Ermittlungsmodell bzw. das Künstliche Neuronale Netz mittels unbeaufsichtigtem Lernen, bestärkendem Lernen oder stochastischem Lernen zu trainieren.

Bevorzugt werden dem Beschichtungszusammensetzungseigenschafts-Ermittlungsmodell bzw. dem (künstlichen) neuronalen Netzwerk die (zu verarbeitenden) Daten, insbesondere der erste und der mindestens eine weitere Analysedatensatz, (oder hierfür charakteristische oder hiervon abgeleitete Daten) als Eingangsgrößen zugeführt. Bevorzugt bildet das Beschichtungszusammensetzungseigenschafts-Ermittlungsmodell bzw. das künstliche neuronale Netzwerk die Eingangsgrößen in Abhängigkeit einer parametrierbaren Verarbeitungskette auf Ausgangsgrößen ab.

Bevorzugt ist als Ausgangsgröße wenigstens eine Beschichtungszusammensetzungseigenschaft und/oder eine für eine Beschichtungszusammensetzungseigenschaft charakteristische Größe gewählt.

Bevorzugt umfasst die Ausgangsgröße auch Daten, die eine erste Beschichtungszusammensetzungskomponente und wenigstens eine weitere Beschichtungszusammensetzungskomponente und vorzugsweise auch deren jeweilige Anteilsgrößen und/oder dafür charakteristische Größen umfasst.

Bevorzugt wird/wurde das Beschichtungszusammensetzungseigenschafts-Ermittlungsmodell maschinellen Lernens unter Verwendung vorgegebener Trainingsdaten trainiert, wobei durch das Training die parametrierbare Verarbeitungskette parametriert wird.

Bei einem bevorzugten Verfahren werden in dem Trainingsprozess des Beschichtungszusammensetzungseigenschafts-Ermittlungsmodells Trainingsdaten verwendet, welche historisch erfasste Daten umfassen, insbesondere für Rezepturen von Beschichtungszusammensetzungen und mindestens eine physiko-chemischen Beschichtungszusammensetzungseigenschaft. Dies bietet den Vorteil, dass in kurzer Zeit eine hohe Zahl an Trainingsdaten erzeugt werden können.

Bevorzugt umfassen die Trainingsdaten als Klassifizierungsmerkmal die Information, ob eine Analysegröße einen Sollwert einnimmt (beziehungsweise innerhalb eines Sollwertbereichs liegt) und/oder ob eine Analysegröße einen Sollwert nicht einnimmt (beziehungsweise außerhalb eines Sollwertbereichs liegt).

Bei einem weiter bevorzugten Verfahren wird die ermittelte Eigenschaftsgröße zur Übermittlung an eine Ausgabeeinrichtung, beispielsweise einen Bildschirm und/oder ein (insbesondere mobiles) Endgerät eines Nutzers gesendet. Ergänzend oder alternativ dazu könnte die ermittelte Eigenschaftsgröße zum Ablegen auf einer (bzw. der obig beschriebenen) externen und/oder Cloud-basierten Speichereinrichtung (insbesondere mit einer (erzeugten) der Beschichtungszusammensetzung zugeordneten Beschichtungszusammensetzungs-ID und/oder Beschichtungszusammensetzungsidentifikationsgröße) bereitgestellt werden und bevorzugt zu einem späteren Zeitpunkt an einen Nutzer mittels einer Ausgabeeinrichtung ausgegeben werden.

Bevorzugt liegt für jede hergestellte, bevorzugt für jede Beschichtungszusammensetzung eine einzigartige Beschichtungszusammensetzungsidentifikationsgröße bzw. Beschichtungszusammensetzungskennnummer (Beschichtungszusammensetzungs-ID) vor, wie obig bereits beschrieben. Die bei der Herstellung der Beschichtungszusammensetzung (insbesondere typischerweise ohnehin standardmäßig) ermittelten bzw. erzeugten Analysedatensätze sind bevorzugt zusammen mit der Beschichtungszusammensetzungsidentifikationsgröße auf der (obig beschriebenen) Datenbank (bevorzugt auf einem in Bezug auf die Beschichtungszusammensetzungseigenschafts-Ermittlungsvorrichtung) externen Server) abgelegt.

Weiterhin ist die Erfindung gerichtet auf ein Computer-implementiertes Verfahren zur Erzeugung eines trainierten Beschichtungszusammensetzungseigenschafts-Ermittlungsmodells maschinellen Lernens zur Ermittlung wenigstens einer physiko-chemischen Beschichtungszusammensetzungseigenschaft einer Beschichtungszusammensetzung auf Basis einer Verwendung von Analysedatensätzen. Diese Analysedatensätze sind jeweils für eine Beschichtungszusammensetzung charakteristisch. Ein Analysedatensatz zu der jeweiligen Beschichtungszusammensetzung umfasst jeweils wenigstens eine Analysegröße und eine Anteilsgröße. Die Analysegröße ist dabei jeweils charakteristisch für wenigstens eine vorgegebene Funktionalität der jeweiligen Beschichtungszusammensetzung und die Anteilsgröße jeweils charakteristisch für einen Mengenanteil der in der jeweiligen Beschichtungszusammensetzung befindlichen ersten und/oder weiteren Beschichtungszusammensetzungskomponente. Das Verfahren umfasst insbesondere die folgenden Schritte:

- Bereitstellen eines trainierbaren Beschichtungszusammensetzungseigenschafts-Ermittlungsmodells maschinellen Lernens, welches einen Satz trainierbarer Parameter umfasst und welches auf Grundlage von zu mindestens einem zu einer Beschichtungszusammensetzung erfassten Analysedatensatz oder hiervon abgeleiteter Daten als Eingangsgrößen wenigstens eine für die physiko-chemische Beschichtungszusammensetzungseigenschaft der Beschichtungszusammensetzung charakteristische Eigenschaftsgröße als Ausgangsgröße erzeugt;

- Erzeugen eines Trainingsdatensatzes umfassend eine Vielzahl erfasster und/oder ermittelter Analysedatensätze zu Trainings-Beschichtungszusammensetzungen, welche zum Erzeugen der Trainingsdatensätzen verwendet werden, sowie, insbesondere mittels einer Vollanalyse der jeweiligen Trainings-Beschichtungszusammensetzung ermittelte, Messdaten oder eine hiervon abgeleitete Messgröße beziehungsweise Analysegröße, welche charakteristisch sind bzw. ist für wenigstens eine physiko-chemische Beschichtungszusammensetzungseigenschaft der jeweiligen Trainings-Beschichtungszusammensetzung;

- Trainieren des Beschichtungszusammensetzungseigenschafts-Ermittlungsmodells auf Grundlage des Trainingsdatensatzes.

Bevorzugt wird das so erzeugte Beschichtungszusammensetzungseigenschafts-Ermittlungsmodell zur Ermittlung wenigstens einer für eine Beschichtungszusammensetzungseigenschaft einer Beschichtungszusammensetzung charakteristischen Eigenschaftsgröße verwendet. Dies kann bevorzugt im Rahmen eine oben beschriebenen Verfahrens erfolgen und/oder durch eine wie im Folgenden beschriebene Beschichtungszusammensetzungseigenschafts-Ermittlungsvorrichtung.

Die vorliegende Erfindung ist weiterhin gerichtet auf eine, bevorzugt Prozessor-basierte, Beschichtungszusammensetzungseigenschafts-Ermittlungsvorrichtung zur Ermittlung wenigstens einer physiko-chemischen Beschichtungszusammensetzungseigenschaft einer Beschichtungszusammensetzung.

Erfindungsgemäß ist die Beschichtungszusammensetzungseigenschafts-Ermittlungsvorrichtung dazu geeignet und bestimmt, einen ersten Analysedatensatzes zu einer zweiten Beschichtungszusammensetzung und mindestens einen weiteren Analysedatensatzes zu mindestens einer dritten Beschichtungszusammensetzung zu erfassen, wobei der Analysedatensatz jeweils wenigstens eine Analysegröße und eine Anteilsgröße einer Beschichtungszusammensetzungskomponente und bevorzugt einer Vielzahl von Anteilsgrößen von weiteren Beschichtungszusammensetzungskomponenten umfasst, wobei die Analysegröße jeweils charakteristisch ist für wenigstens eine vorgegebene Funktionalität der jeweiligen Beschichtungszusammensetzung, und wobei die Anteilsgröße jeweils charakteristisch ist für einen Mengenanteil der in der jeweiligen Beschichtungszusammensetzung befindlichen ersten und/oder weiteren Beschichtungszusammensetzungskomponenten.

Dabei ist die Beschichtungszusammensetzungseigenschafts-Ermittlungsvorrichtung dazu geeignet und bestimmt, mittels einer Prozessoreinrichtung wenigstens eine für die Beschichtungszusammensetzungseigenschaft der ersten Beschichtungszusammensetzung, charakteristische physiko-chemischen Eigenschaftsgröße auf Grundlage des Analysedatensatzes zu der zweiten Beschichtungszusammensetzung und des mindestens einen weiteren Analysedatensatzes zu der mindestens einen dritten Beschichtungszusammensetzung zu ermitteln.

Bevorzugt ist der Analysedatensatz jeweils entsprechend einer obig beschriebenen (bevorzugten) Ausführungsform ausgestaltet.

Es wird also auch im Rahmen der erfindungsgemäßen Beschichtungszusammensetzungseigenschafts-Ermittlungsvorrichtung vorgeschlagen, dass durch die Verwendung der Analysedatensätze bereits weitegehend vorliegende (und gegebenenfalls durch Analysedaten ergänzte) Datensätze verwendet werden, insbesondere solche, welche standardmäßig bei der Herstellung von Beschichtungszusammensetzung bereitgestellt werden. Die Menge an zu übermittelnden Daten ist gegenüber den Einzelkomponenten erheblich kleiner. Durch die obig im Rahmen des Verfahrens beschriebenen Verwendung von funktional aggregierten Gruppen wird vorteilhaft ein robusteres Ergebnis erzeugt.

Bevorzugt ist die Beschichtungszusammensetzungseigenschafts-Ermittlungsvorrichtung dazu eingerichtet, geeignet und/oder bestimmt, das obig beschriebene Verfahren zur Ermittlung wenigstens einer physiko-chemischen Beschichtungszusammensetzungseigenschaft einer ersten Beschichtungszusammensetzung sowie alle bereits obig im Zusammenhang mit dem Verfahren (oder einer bevorzugten Ausführungsform des Verfahrens) beschriebene Verfahrensschritte einzeln oder in Kombination miteinander auszuführen. Umgekehrt kann das Verfahren mit allen im Rahmen der Beschichtungszusammensetzungseigenschafts-Ermittlungsvorrichtung beschriebenen Merkmalen einzeln oder in Kombination miteinander ausgestattet sein.

Weiterhin ist die Beschichtungszusammensetzungseigenschafts-Ermittlungsvorrichtung bevorzugt dazu geeignet und bestimmt, ein gemäß obigem Verfahren beschriebenes erzeugtes trainiertes Beschichtungszusammensetzungseigenschafts-Ermittlungsmodell (bevorzugt in Bezug auf die erste Beschichtungszusammensetzung) zu verwenden. Bevorzugt wird hierzu dem Beschichtungszusammensetzungseigenschafts-Ermittlungsmodell ein erster Analysedatensatz und mindestens ein weiterer Analysedatensatz zugeführt. Hierzu ist die Beschichtungszusammensetzungseigenschafts-Ermittlungsvorrichtung bevorzugt dazu geeignet und bestimmt, aus den dem Beschichtungszusammensetzungseigenschafts-Ermittlungsmodell zuzuführenden Analysedatensätzen eine Beschichtungszusammensetzung zu ermitteln, welche die wenigsten eine physiko-chemische Beschichtungszusammensetzungseigenschaft aufweist.

Unter einem obig beschriebenen externen Server ist insbesondere ein in Bezug auf die Beschichtungszusammensetzungseigenschafts-Ermittlungsvorrichtung gesehener externer Server, insbesondere ein Backend-Server, zu verstehen. Der externe Server ist beispielsweise ein Backend eines Produzenten für Beschichtungen und/oder eines Händlers für Beschichtungen, Rohstoffanbieter für Produkte in der Beschichtungsindustrie oder eines Dienstanbieters, welcher dazu eingerichtet ist, Analysedatensätze in Bezug auf Beschichtungszusammensetzungen zu ermitteln und/oder zu erzeugen und/oder zu verwalten und/oder zu speichern. Die Funktionen des Backend bzw. des externen Servers können dabei auf (externen) Serverfarmen durchgeführt werden. Beim (externen) Server kann es sich um ein verteiltes System handeln. Der externe Server und/oder das Backend kann Cloud-basiert sein.

Die vorliegende Erfindung ist weiterhin gerichtet auf eine Anlage zur Herstellung einer Beschichtungszusammensetzung, umfassend eine obig beschriebene Beschichtungszusammensetzungseigenschafts-Ermittlungsvorrichtung entsprechend einer (obig beschriebenen) Ausführungsform.

Bevorzugt ist die Anlage mit einer entsprechenden Schnittstelle ausgerüstet, welche die Übermittlung der Analysedatensätze und/oder die Auswahl einer gewünschten Beschichtungszusammensetzungseigenschaft ermöglicht.

Die vorliegende Erfindung ist weiterhin gerichtet auf ein Computerprogramm oder Computerprogrammprodukt, umfassend Programmmittel, insbesondere einen Programmcode, welcher zumindest einige der und bevorzugt alle Verfahrensschritte der erfindungsgemäßen Verfahren (Verfahren zur Ermittlung wenigstens einer physiko-chemischen Beschichtungszusammensetzungseigenschaft einer ersten Beschichtungszusammensetzung; Verfahren zur Erzeugung eines trainierten Beschichtungszusammensetzungseigenschafts-Ermittlungsmodells maschinellen Lernens; Verwendung des erzeugten trainierten Beschichtungszusammensetzungseigenschafts-Ermittlungsmodells maschinellen Lernens zur Ermittlung wenigstens einer physiko-chemischen Beschichtungszusammensetzungseigenschaft einer ersten Beschichtungszusammensetzung) und bevorzugt eine der beschriebenen bevorzugten Ausführungsformen repräsentiert oder kodiert und zum Ausführen durch eine Prozessoreinrichtung ausgebildet ist.

Die vorliegende Erfindung ist weiterhin gerichtet auf einen Datenspeicher, auf welchem zumindest eine Ausführungsform des erfindungsgemäßen Computerprogramms oder einer bevorzugten Ausführungsform des Computerprogramms gespeichert ist.

Die vorliegende Erfindung wurde in Bezug auf eine Beschichtungszusammensetzung beschrieben, wobei dieser Begriff üblicherweise auf ein Objekt aufgebracht werden, um diesem Objekt eine gewünschte Oberflächeneigenschaft, beispielsweise einen gewünschten Farbort zu verleihen. Dabei ist die vorliegende Erfindung vorallem anwendbar auf Beschichtungen allgemein, deren physiko-chemischen Eigenschaft dem beschichteten Objekt eine gewünschte Eigenschaft verleiht. Denkbar wäre dabei eine Schutzbeschichtung, insbesondere eine Korrosionsschutzbeschichtung, eine schalldämpfende Beschichtung, eine (insbesondere elektromagnetische Wellen) reflektierende oder absorbierende Beschichtung oder eine Beschichtung zur Veränderung der Haptik des Objekts. Die Anmelderin behält sich vor, hierauf gerichtete Gegenstände ebenfalls zu beanspruchen.

Weitere Vorteile und Ausführungsformen ergeben sich aus der beigefügten Zeichnung:

Darin zeigt:

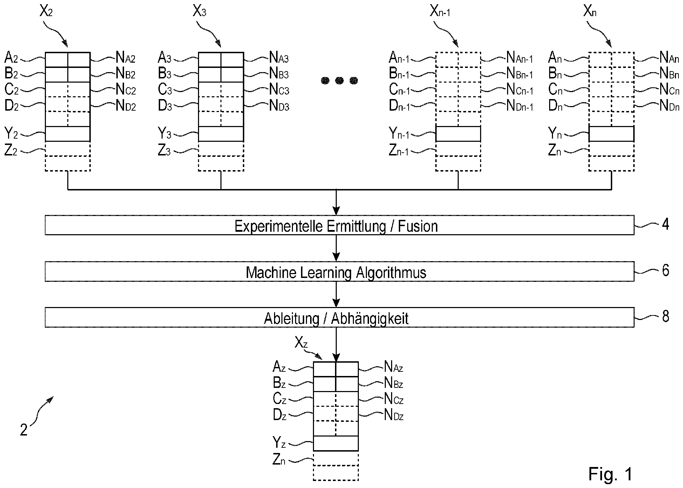

Fig. 1 eine schematische Darstellung eines Ablaufs eines erfindungsgemäßen Verfahrens gemäß einer bevorzugten Ausführungsform.

Fig. 1 zeigt eine schematische Darstellung eines Ablaufs eines erfindungsgemäßen Verfahrens (2) gemäß einer bevorzugten Ausführungsform. Die Bezugszeichen $X_2$ - $X_n$ kennzeichnen jeweils einen Analysedatensatz, welcher Deskriptoren für jeweils eine Beschichtungszusammensetzung umfasst. Diese Deskriptoren können durch Analyse erhalten werden oder aus der Rezeptur der jeweiligen Beschichtungszusammensetzung bekannt sein.

Die Deskriptoren für jeweils eine Beschichtungszusammensetzung schließen mindestens eine erste Beschichtungszusammensetzungskomponente ($A_X$ (mit $x \in \{\mathbb{N} \geq 2, \leq n\}$)) und mindestens eine weitere Beschichtungszusammensetzungskomponente ($B_X$ (ebenfalls mit $x \in \{\mathbb{N} \geq 2, \leq n\}$)) sowie die diesen Beschichtungszusammensetzungskomponenten zugehörigen Anteilsgrößen ($N_{Ax}$ und $N_{Bx}$) ein. Weitere Beschichtungszusammensetzungskomponenten ($C_X$, $D_X$, ...) und deren jeweiligen Anteile ($N_{Cx}$, $N_{Dx}$, ...) in der Beschichtungszusammensetzung sind lediglich durch gestrichelte Bereiche angedeutet.

Die Anzahl der Analysedatensätze $X_2$ - $X_n$ ist nach oben offen. Eine große Anzahl ist jedoch vorteilhaft, um die Robustheit des Verfahrens zu verbessern. Es hat sich aber gezeigt, dass bereits ein Paket von mehr als 50 Analysedatensätzen (n = 50) ausreichend sein kann, um annehmbar gute Ergebnisse zu erreichen. Eine größere Anzahl von Analysedatensätzen wie beispielsweise $\geq$ 150 Analysedatensätze (n $\geq$ 150) ist jedoch wie oben dargelegt vorteilhaft im Hinblick auf die Qualität und Robustheit einer Berechnung beziehungsweise Vorhersage einer Beschichtungszusammensetzungseigenschaft.

Jedem Analysedatensatz ist mindestens eine Analysegröße $Y_2$ - $Y_n$ zugeordnet. Mehrere Analysegrößen sind wie oben dargelegt vorteilhaft, wobei in Fig. 1 für jeden Analysedatensatz lediglich eine weitere Analysegröße ($Z_X$ (mit $x \in \{\mathbb{N} \geq 2, \leq n\}$)) bezeichnet und diese weiteren Analysegrößen lediglich durch gestrichelte Bereiche angedeutet sind.

Eine Analysegröße ($Y_X, Z_X, ...$) steht jeweils für eine Beschichtungszusammensetzungseigenschaft. Beispielsweise kann es sich dabei um einen Farbort, eine Viskosität, ein Deckvermögen, eine Abriebbeständigkeit oder eine andere der oben genannten Eigenschaften handeln. Diese Eigenschaft kann bereits zusammen mit dem Analysedatensatz ($X_X$ (mit $x \in \{\mathbb{N} \geq 2, \leq n\}$)) bereitgestellt werden oder diesen nachträglich, beispielsweise nach einer entsprechenden Analyse der betreffenden Beschichtungszusammensetzung zugefügt werden. Es ist dabei jedoch nicht notwendig, dass jeder Analysedatensatz ($X_2$ - $X_n$) zu jeder Beschichtungszusammensetzungseigenschaft ($Y_X, Z_X, ...$) ein Datum enthält. Es ist auch denkbar, dass einzelne der Analysedatensätze ($X_2$ - $X_n$) zu einer bestimmten Beschichtungszusammensetzungseigenschaft ($Y_X, Z_X, ...$) kein Datum zugeordnet ist.

Es wird vorgeschlagen, die Analysedatensätze ($X_2$ - $X_n$) beziehungsweise die darin enthaltenen Deskriptoren ($A_X, B_X, ...; N_{Ax}, N_{Bx}, ...$) einschließlich der Analysegröße $Y_2$ - $Y_n$ für einen Machine-Learning-Algorithmus (6) zu verwenden. Hierzu ist in dem mit dem Bezugzeichen (4) gekennzeichneten Verfahrensschritt eine Experimentelle Ermittlung/Fusion der Analysedatensätze ($X_2$ - $X_n$) und der Analysegröße ($Y_2$ - $Y_n$) vorgesehen.

Mit einer Anzahl an Trainingsdatensätzen wird nun der Machine-Learning-Algorithmus (6) trainiert und für den jeweiligen Zielwert, welcher zu einer Beschichtungszusammensetzungseigenschaft ($Y_Z$) korreliert, eine Ableitungsfunktion in Abhängigkeit von den ermittelten Parametern erzeugt. In Fig. 1 kennzeichnet das Bezugszeichen (8) die Ableitung einer derartigen Abhängigkeit.

Der Trainingsdatensatz wird zum Training des Machine-Learning-Algorithmus (6) bzw. zum Trainieren eines neuronalen Netzwerkes bzw. zum maschinellen Lernen zur Vorhersage der physiko-chemischen Beschichtungszusammensetzungseigenschaft ($Y_Z$) als Funktion der Analysedatensätze ($X_2$ - $X_n$) verwendet. Hieraus (durch das trainierte neuronale Netz bzw. durch den trainierten Machine-Learning-Algorithmus) ergibt sich insbesondere eine Vorhersagefunktion, welche bevorzugt zur Vorhersage wenigstens einer physiko-chemischen Beschichtungszusammensetzungseigenschaft ($Y_Z$) einer ersten Beschichtungszusammensetzung ($X_Z$) verwendet werden kann. Aus dem zu der ersten Beschichtungszusammensetzung korrelierenden Analysedatensatz ($X_Z$) und der Vorhersagefunktion kann insbesondere ein Erwartungswert zu der wenigstens einen physiko-chemischen Beschichtungszusammensetzungseigenschaft ($Y_Z$) dieser ersten Beschichtungszusammensetzung ($X_Z$) ermittelt werden.

Ebenso ist es denkbar, einen Soll-Wert der physiko-chemischen Beschichtungszusammensetzungseigenschaft ($Y_Z$) vorzugeben und das System Deskriptoren für jeweils eine Beschichtungszusammensetzung ermitteln zu lassen welche gemäß dieser Vorhersage wahrscheinlich die vorgegebene Beschichtungszusammensetzungseigenschaft ($Y_Z$) aufweist. Bei dieser Variante des Verfahrens wird somit ein Soll-Wert der physiko-chemischen Beschichtungszusammensetzungseigenschaft ($Y_Z$) vorgegeben und die Beschichtungszusammensetzungskomponenten ($A_Z$, $B_Z$, $C_Z$,...) sowie die diesen Beschichtungszusammensetzungskomponenten zugehörigen Anteilsgrößen ($N_{Az}$, $N_{Bz}$, $N_{Cz}$, ...) ermittelt. Eine Beschichtungszusammensetzung mit den Beschichtungszusammensetzungskomponenten ($A_Z$, $B_Z$, $C_Z$,...) in den Mengenanteilen gemäß der Anteilsgrößen ($N_{Az}$, $N_{Bz}$, $N_{Cz}$, ...) wird gemäß dieser Vorhersage die vorgegebene physiko-chemischen Beschichtungszusammensetzungseigenschaft ($Y_Z$) mit dem Soll-Wert aufweisen.

Die Anmelderin behält sich vor, sämtliche in den Anmeldungsunterlagen offenbarten Merkmale als erfindungswesentlich zu beanspruchen, sofern sie einzeln oder in Kombination gegenüber dem Stand der Technik neu sind. Es wird weiterhin darauf hingewiesen, dass in der Figur auch Merkmale beschrieben wurden, welche für sich genommen vorteilhaft sein können. Der Fachmann erkennt unmittelbar, dass ein bestimmtes in der Figur beschriebenes Merkmal auch ohne die Übernahme weiterer Merkmale aus dieser Figur vorteilhaft sein kann. Ferner erkennt der Fachmann, dass sich auch Vorteile durch eine Kombination eines oder mehrerer in einer Figur gezeigter Merkmale mit einem oder mehreren im Text beschriebener Merkmale ergeben können.

## Patentansprüche

1. Verfahren zur, insbesondere prädiktiven, Ermittlung wenigstens einer physiko-chemischen Beschichtungszusammensetzungseigenschaft ($Y_Z$) einer ersten Beschichtungszusammensetzung ($X_Z$), welches eine erste Beschichtungszusammensetzungskomponente ($A_Z$) und wenigstens eine weitere, von der ersten Beschichtungszusammensetzungskomponente ($A_Z$) verschiedene Beschichtungszusammensetzungskomponente ($B_Z$) umfasst, mit den Schritten:

    - Erfassung eines ersten Analysedatensatzes ($X_2$) zu einer zweiten Beschichtungszusammensetzung, wobei der erste Analysedatensatz ($X_2$) wenigstens eine Analysegröße ($Y_2$) und eine Anteilsgröße ($N_{A2}$) einer ersten Beschichtungszusammensetzungskomponente ($A_2$) und bevorzugt einer Vielzahl von Anteilsgrößen ($N_{B2}$, $N_{C2}$,...) von weiteren Beschichtungszusammensetzungskomponenten ($B_2$, $C_2$,...) umfasst,

    - Erfassung mindestens eines weiteren Analysedatensatzes ($X_3$, ... $X_n$) zu mindestens einer dritten Beschichtungszusammensetzung, wobei der mindestens eine weitere Analysedatensatz ($X_3$) wenigstens eine Analysegröße ($Y_3$) und eine Anteilsgröße ($N_{A3}$) einer ersten Beschichtungszusammensetzungskomponente ($A_3$) und bevorzugt einer Vielzahl von Anteilsgrößen ($N_{B3}$, $N_{C3}$,...) von weiteren Beschichtungszusammensetzungskomponenten ($B_3$, $C_3$, ...) umfasst,

    - wobei die Analysegröße ($Y_A$ - $Y_n$) jeweils charakteristisch ist für wenigstens eine Beschichtungszusammensetzungseigenschaft der durch den jeweiligen Analysedatensatz ($X_2$, $X_3$,... $X_n$) charakterisierten Beschichtungszusammensetzung,

    - wobei die Anteilsgröße ($N_{XY}$) jeweils charakteristisch ist für einen Mengenanteil der in der durch den jeweiligen Analysedatensatz ($X_2$, $X_3$,...,$X_n$) charakterisierten Beschichtungszusammensetzung befindlichen ersten und/oder weiteren Beschichtungszusammensetzungskomponenten ($A_2$, $A_3$,...,$A_n$; $B_2$, $B_3$,...,$B_n$;...); und

    - Computer-implementierte Ermittlung von wenigstens einer für die Beschichtungszusammensetzungseigenschaft ($Y_Z$, $Z_Z$,...) der ersten Beschichtungszusammensetzung ($X_Z$) charakteristischen physiko-chemischen Eigenschaftsgröße auf Grundlage des Analysedatensatzes ($X_2$) zu der zweiten Beschichtungszusammensetzung und des mindestens einen weiteren Analysedatensatzes ($X_3$, ... $X_n$) zu der mindestens einen dritten Beschichtungszusammensetzung.

2. Verfahren nach Anspruch 1, wobei die zu ermittelnde wenigstens eine Beschichtungszusammensetzungseigenschaft ($Y_Z$, $Z_Z$,...) ausgewählt ist aus einer Gruppe, die einen Abrasionswiderstand, einen Nassabriebbeständigkeit, ein Deckvermögen, einen Farbwert (und/oder Farbort), einen Gelbwert, einen Weißgrad, einen Glanz, eine Dichte, eine Auftragsmenge, eine Ausbeute, eine Ergiebigkeit, eine Viskosität, einen Feststoffanteil, einen Lösungsmittelanteil, eine Wasserlöslichkeit, eine Wasseremulgierbarkeit, eine Wassersuspendierbarkeit, eine UV-Beständigkeit, eine Temperaturwiderstandfähigkeit, einen Lagerfähigkeit, eine Diffusionseigenschaft, eine Atmungsaktivät, einen Widerstandsfähigkeit, einen pH-Wert, einen Schadstoffwert, eine Hitzewiderstandsfähigkeit, eine Feuerfestigkeitsfähigkeit, eine angemessene Entsorgungsmöglichkeit für die Beschichtungszusammensetzung und ein Gesundheitsgefährdungsrisiko umfasst.

3. Verfahren nach einem der vorhergehenden Ansprüche, wobei es sich bei der ersten Beschichtungszusammensetzungskomponente ($A_y$) und der wenigstens einen weiteren Beschichtungszusammensetzungskomponente ($B_X$) jeweils um ein Additiv handelt.

4. Verfahren nach einem der vorangegangenen Ansprüche, wobei die erste Beschichtungszusammensetzungskomponente ($A_y$) und/oder wenigstens eine weitere Beschichtungszusammensetzungskomponente ($B_y$) ausgewählt ist aus einer Gruppe, die Füllstoff, Rheologieadditiv, Pigment, Farbstoff, Emulgator, Biozid, und Konservierungsmittel umfasst, wobei die erste Beschichtungszusammensetzungskomponente ($A_y$) und/oder wenigstens eine weitere Beschichtungszusammensetzungskomponente ($B_y$) bevorzugt ein Kaolin- und/oder Kaolinfolgeprodukt und/oder eine Ca- und/oder Mg-haltige Komponente ist.

5. Verfahren nach einem der vorangegangenen Ansprüche, wobei die Analysegröße ($Y_y$) charakteristisch ist für eine Eigenschaft, die ausgewählt ist aus einer Gruppe, die einen Abrasionswiederstand, einen Nassabriebbeständigkeit, eine Deckeigenschaft, einen Farbwert, einen Gelbwert, eine Weißgrad, einen Glanz, eine Dichte, eine Auftragsmenge, eine Ausbeute, eine Ergiebigkeit, eine Viskosität, einen Feststoffanteil, einen Lösungsmittelanteil, eine Wasserlöslichkeit, eine Wasseremulgierbarkeit, eine Wassersuspendierbarkeit, eine UV-Beständigkeit, eine Temperaturwiderstandfähigkeit, einen Lagerfähigkeit, eine Diffusionseigenschaft, eine Atmungsaktivität, einen Widerstandsfähigkeit, einen pH-Wert, einen Schadstoffwert, eine Hitzewiderstandsfähigkeit, eine Feuerfestigkeitsfähigkeit, eine angemessene Entsorgungsmöglichkeit für die Beschichtungszusammensetzung und ein Gesundheitsgefährdungsrisiko umfasst.

6. Verfahren nach einem der vorangegangenen Ansprüche, wobei der erste Analysedatensatz ($X_2$) durch eine Analyse, vorzugsweise eine Vollanalyse, der zweiten Beschichtungszusammensetzung erhalten wird und/oder der zweite Analysedatensatz ($X_3, ... X_n$) durch eine Analyse, vorzugsweise eine Vollanalyse, der weiteren Beschichtungszusammensetzung erhalten wird.

7. Verfahren nach einem der vorangegangenen Ansprüche, wobei die Ermittlung der wenigstens einen physio-chemischen Beschichtungszusammensetzungseigenschaft ($Y_Z, Z_Z;...$) der ersten Beschichtungszusammensetzung ($X_Z$) auf Grundlage von zu einer Vielzahl verschiedener Beschichtungszusammensetzungen jeweils erfassten Analysedatensätzen ($X_2 - X_n$) erfolgt.

8. Verfahren nach einem der vorangegangenen Ansprüche, **dadurch gekennzeichnet, dass** auf Grundlage eines Soll-Wertes wenigstens einer und bevorzugt einer Vielzahl von Eigenschaftsgrößen ($Y_Z, Z_Z,...$) der Beschichtungszusammensetzung eine der Beschichtungszusammensetzung zuzuführende erste Beschichtungszusammensetzungskomponente ($A_Z$) und deren Anteilsgröße ($N_{AZ}$) ermittelt wird und/oder eine der Beschichtungszusammensetzung zuzuführende weitere Beschichtungszusammensetzungskomponente ($B_Z, C_Z, ...$) und deren jeweilige Anteilsgröße ($N_{Bz}, N_{Cz},...$) ermittelt wird.

9. Ein Computer-implementiertes Verfahren zur Erzeugung eines trainierten Beschichtungszusammensetzungseigenschafts-Ermittlungsmodells maschinellen Lernens (6) zur Ermittlung wenigstens einer physiko-chemischen Beschichtungszusammensetzungseigenschaft ($Y_Z, Z_Z,...$) einer Beschichtungszusammensetzung auf Basis einer Verwendung von Analysedatensätzen ($X_2 - X_n$), welche jeweils für eine Beschichtungszusammensetzung charakteristisch sind, wobei der Analysedatensatz ($X_2 - X_n$) zu der jeweiligen Beschichtungszusammensetzung jeweils wenigstens eine Analysegröße ($Y_2, Y_3,..., Y_n$) und eine Anteilsgröße ($N_{XY}$) umfasst, wobei die Analysegröße jeweils charakteristisch ist für wenigstens eine vorgegebene Funktionalität der jeweiligen Beschichtungszusammensetzung ($X_2, X_3,..., X_n$) und wobei die Anteilsgröße ($N_{XY}$) jeweils charakteristisch ist für einen Mengenanteil der in der jeweiligen Beschichtungszusammensetzung befindlichen ersten und/oder weiteren Beschichtungszusammensetzungskomponente ($A_2, A_3,...,A_n, B_2, B_3,...,B_n,...$), umfassend:

- Bereitstellen eines trainierbaren Beschichtungszusammensetzungseigenschafts-Ermittlungsmodells (6) maschinellen Lernens, welches einen Satz trainierbarer Parameter umfasst und welches auf Grundlage von zu mindestens einem zu einer Beschichtungszusammensetzung erfassten Analysedatensatz ($X_2 - X_n$) oder hiervon abgeleiteter Daten als Eingangsgrößen wenigstens eine für die physiko-chemische Beschichtungszusammensetzungseigenschaft der Beschichtungszusammensetzung charakteristische Eigenschaftsgröße ($Y_Z$) als Ausgangsgröße erzeugt;
- Erzeugen eines Trainingsdatensatzes umfassend eine Vielzahl erfasster und/oder ermittelter Analysedatensätze ($X_2 - X_n$) zu Trainings-Beschichtungszusammensetzungen, welche zum Erzeugen der Trainingsdatensätzen verwendet werden, sowie, insbesondere mittels einer Vollanalyse der jeweiligen Trainings-Beschichtungszusammensetzung ermittelte, Messdaten oder eine hiervon abgeleitete Messgröße (Ax - Zx), welche charakteristisch sind bzw. ist für wenigstens eine physiko-chemische Beschichtungszusammensetzungseigenschaft der jeweiligen Trainings-Beschichtungszusammensetzung;

- Trainieren des Beschichtungszusammensetzungseigenschafts-Ermittlungsmodells auf Grundlage des Trainingsdatensatzes.

10. Verwendung des nach dem vorhergehenden Anspruch erzeugten Beschichtungszusammensetzungseigenschafts-Ermittlungsmodells (6) zur Ermittlung wenigstens einer für eine Beschichtungszusammensetzungseigenschaft einer Beschichtungszusammensetzung charakteristischen Eigenschaftsgröße $(Y_Z)$.

11. Beschichtungszusammensetzungseigenschafts-Ermittlungsvorrichtung (10) zur Ermittlung wenigstens einer physiko-chemischen Beschichtungszusammensetzungseigenschaft $(Y_Z)$ einer Beschichtungszusammensetzung,

**dadurch gekennzeichnet, dass**

die Beschichtungszusammensetzungseigenschafts-Ermittlungsvorrichtung (10) dazu geeignet und bestimmt ist, einen ersten Analysedatensatz $(X_2)$ zu einer zweiten Beschichtungszusammensetzung und mindestens einen weiteren Analysedatensatz $(X_3, ... X_n)$ zu mindestens einer dritten Beschichtungszusammensetzung zu erfassen, wobei der Analysedatensatz $(X_2, ... X_n)$ jeweils wenigstens eine Analysegröße $(Y_2, ..., Y_n)$ und eine Anteilsgröße $(N_{A2})$ einer Beschichtungszusammensetzungskomponente $(A_2)$, und bevorzugt einer Vielzahl von Anteilsgrößen $(N_{XY})$ von weiteren Beschichtungszusammensetzungskomponenten $(B_2, B_3,...,B_n, C_2, C_3,..., C_n, ...)$, umfasst, wobei die Analysegröße $(Y_2, ..., Y_n)$ jeweils charakteristisch ist für wenigstens eine vorgegebene Funktionalität der jeweiligen Beschichtungszusammensetzung, und wobei die Anteilsgröße $(N_{XY})$ jeweils charakteristisch ist für einen Mengenanteil der in der jeweiligen Beschichtungszusammensetzung befindlichen ersten und/oder weiteren Beschichtungszusammensetzungskomponenten $(A_2, A_3,...,A_n, B_2, B_3,...,B_n)$, wobei die Beschichtungszusammensetzungseigenschafts-Ermittlungsvorrichtung (10) dazu geeignet und bestimmt ist, mittels einer Prozessoreinrichtung wenigstens eine für die Beschichtungszusammensetzungseigenschaft der ersten Beschichtungszusammensetzung, charakteristische physiko-chemischen Eigenschaftsgröße $(Y_Z)$ auf Grundlage des Analysedatensatzes $(X_2)$ zu der zweiten Beschichtungszusammensetzung und des mindestens einen weiteren Analysedatensatzes $(X_3, ... X_n)$ zu der mindestens einen dritten Beschichtungszusammensetzung zu ermitteln.

Fig. 1

Europäisches
Patentamt

European
Patent Office

Office européen
des brevets

# EUROPÄISCHER RECHERCHENBERICHT

Nummer der Anmeldung

EP 25 18 6613

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2024/184952 A1 (THOMAS MARC [DE] ET AL) 6. Juni 2024 (2024-06-06) * das ganze Dokument * ----- | 1-11 | INV. G16C60/00 |
| A | WO 2024/015832 A1 (PPG IND OHIO INC [US]) 18. Januar 2024 (2024-01-18) * das ganze Dokument * ----- | 1-11 | ADD. G16C20/30 G16C20/70 |
| A | US 2006/031027 A1 (ALMAN DAVID H [US]) 9. Februar 2006 (2006-02-09) * das ganze Dokument * ----- | 1-11 | |
| A | US 2019/078936 A1 (BISCHOFF GUIDO [DE] ET AL) 14. März 2019 (2019-03-14) * das ganze Dokument * ----- | 1-11 | |

RECHERCHIERTE
SACHGEBIETE (IPC)

G16C
G06N

Der vorliegende Recherchenbericht wurde für alle Patentansprüche erstellt

| Recherchenort | Abschlußdatum der Recherche | Prüfer |
|---|---|---|
| Den Haag | 19. Dezember 2025 | Godzina, Przemyslaw |

EPO FORM 1503 03.82 (P04C03)

# EP 4 697 337 A1

| Im Recherchenbericht angeführtes Patentdokument | Datum der Veröffentlichung | Mitglied(er) der Patentfamilie | Datum der Veröffentlichung |
|---|---|---|---|
| US 2024184952 A1 | 06-06-2024 | CN 116997969 A | 03-11-2023 |
| | | EP 4309182 A1 | 24-01-2024 |
| | | JP 2024516501 A | 16-04-2024 |
| | | US 2024184952 A1 | 06-06-2024 |
| | | WO 2022194539 A1 | 22-09-2022 |
| WO 2024015832 A1 | 18-01-2024 | CN 119816900 A | 11-04-2025 |
| | | EP 4555520 A1 | 21-05-2025 |
| | | JP 2025525528 A | 05-08-2025 |
| | | KR 20250040667 A | 24-03-2025 |
| | | WO 2024015832 A1 | 18-01-2024 |
| US 2006031027 A1 | 09-02-2006 | AU 2005271365 A1 | 16-02-2006 |
| | | CA 2571204 A1 | 16-02-2006 |
| | | CN 1993698 A | 04-07-2007 |
| | | EP 1782312 A1 | 09-05-2007 |
| | | JP 2008509486 A | 27-03-2008 |
| | | KR 20070053705 A | 25-05-2007 |
| | | US 2006031027 A1 | 09-02-2006 |
| | | US 2008010027 A1 | 10-01-2008 |
| | | WO 2006017742 A1 | 16-02-2006 |
| US 2019078936 A1 | 14-03-2019 | CN 108139271 A | 08-06-2018 |
| | | DE 102015118551 A1 | 04-05-2017 |
| | | EP 3368872 A1 | 05-09-2018 |
| | | ES 2981196 T3 | 07-10-2024 |
| | | JP 6707637 B2 | 10-06-2020 |
| | | JP 2019500588 A | 10-01-2019 |
| | | KR 20180074756 A | 03-07-2018 |
| | | RU 2702997 C1 | 15-10-2019 |
| | | US 2019078936 A1 | 14-03-2019 |
| | | WO 2017071824 A1 | 04-05-2017 |

EPO FORM P0461